# EUROPEAN PATENT APPLICATION

(11) **EP 4 699 594 A1**
(43) Date of publication of application: **25.02.2026**
(21) Application number: 24792740.3
(22) Date of filing: 18.04.2024
(51) Int. Cl.: A61J 1/20, A61M 5/32

(54) **INJECTION AND MIXING DEVICE**

(30) Priority: 19.04.2023 JP 2023068731; 27.10.2023 JP 2023185021
(71) Applicant: YUYAMA MFG. CO., LTD., Toyonaka-shi, Osaka 561-0841 (JP)
(72) Inventor: UENO, Takashi, Toyonaka-shi Osaka 561-0841 (JP); NOMURA, Keiichi, Toyonaka-shi Osaka 561-0841 (JP); KAWAUCHI, Kazuki, Toyonaka-shi Osaka 561-0841 (JP)
(74) Representative: Plasseraud IP
(86) International application number: PCT/JP2024/015446
(87) International publication number: WO 2024/219468

(57) **Abstract**

In order to release a user from the dangerous work of removing a needle from a discarded syringe, provided is a drug-mixing apparatus (1) including: a drug-mixing unit (40) configured to execute a drug-mixing operation of mixing a drug housed in a vial, into an infusion housed in an infusion container, using a syringe having a needle attached thereto; and a needle removal unit (170) configured to remove the needle from the syringe used in the drug-mixing operation, after the drug-mixing operation has ended.

## Description

### BACKGROUND OF THE INVENTION

### 1. Field of the Invention

The present disclosure relates to a drug-mixing apparatus.

### 2. Description of the Related Art

In Japanese Patent Application Laid-open No. 2019-063313, an example of a drug-mixing apparatus is disclosed. The drug-mixing apparatus of Japanese Patent Application Laid-open No. 2019-063313 includes a drug filling unit and a drug-mixing processing unit. A user places a drug container, members that can form a syringe, and an infusion container, at predetermined positions on a tray placed on a work table of the drug filling unit, and then supplies the tray into the drug-mixing processing unit. Thus, the drug-mixing apparatus of Japanese Patent Application Laid-open No. 2019-063313 can start drug-mixing processing.

### SUMMARY OF THE INVENTION

The drug-mixing apparatus of Japanese Patent Application Laid-open No. 2019-063313 includes a syringe needle attaching/detaching device that attaches and detaches both a needle attached to a syringe and a needle cap to and from a barrel, or attaches and detaches only the needle cap. In the drug-mixing apparatus of Japanese Patent Application Laid-open No. 2019-063313, after the needle cap is attached to the needle of the syringe by the syringe needle attaching/detaching device, the entire syringe is discarded. After that, the user performs an operation of removing the needle from the discarded syringe in order to separately discard the barrel and the like and the needle. A first aspect of the present invention has an object to release a user from the dangerous work of removing a needle from a discarded syringe.

Further, in Japanese Patent Application Laid-open No. 2019-063313, there is no disclosure of specific control of a continuous drug-mixing operation. A second aspect of the present invention has an object to shorten the time required for a continuous drug-mixing operation.

Further, in Japanese Patent Application Laid-open No. 2019-063313, there is no disclosure of a specific method of dissolving a powdered drug in an infusion. A third aspect of the present invention has an object to shorten the time required for a drug-mixing operation for dissolving a powdered drug in an infusion.

Further, in Japanese Patent Application Laid-open No. 2019-063313, there is no disclosure of a mode of gripping the syringe at the time of discarding the syringe into a waste storage chamber. A fourth aspect of the present invention has an object to reduce the possibility that a syringe to be discarded may tilt.

According to a first aspect of the present invention, there is provided a drug-mixing apparatus, including: a drug-mixing unit configured to execute a drug-mixing operation of mixing a drug housed in a drug container into an infusion housed in an infusion container, using a syringe having a needle attached thereto; and a needle removal part configured to remove the needle from the syringe used in the drug-mixing operation, after the drug-mixing operation has ended.

According to a second aspect of the present invention, there is provided a drug-mixing apparatus, including: a drug-mixing unit configured to dissolve, based on a prescription data group including a plurality of pieces of prescription data, a powdered drug housed in a drug container, with an infusion, and execute a drug-mixing operation of mixing the infusion having the powdered drug dissolved therein, into an infusion housed in an infusion container, using a syringe; and an equipment removal unit configured to remove the syringe, the drug container, and the infusion container that have been held by the drug-mixing unit, from the drug-mixing unit. In the drug-mixing operation, the drug-mixing unit waits for a predetermined time after injecting the infusion into the drug container, and then withdraws the infusion from the drug container. When a second drug-mixing operation for second prescription data is to be performed after a first drug-mixing operation for first prescription data, after the equipment removal unit removes the syringe, the drug container, and the infusion container being used in the first drug-mixing operation, from the drug-mixing unit during the waiting for the predetermined time in the first drug-mixing operation, the drug-mixing unit starts the second drug-mixing operation.

According to a third aspect of the present invention, there is provided a drug-mixing apparatus, which is configured to execute a drug-mixing operation of dissolving a powdered drug housed in a drug container, with an infusion, and mixing the infusion having the powdered drug dissolved therein, into an infusion housed in an infusion container. When part of the infusion having the powdered drug dissolved therein is to be withdrawn from the drug container, after executing a dissolving operation of dissolving the powdered drug in the infusion, the drug-mixing apparatus withdraws the infusion having the powdered drug dissolved therein, from the drug container after a first predetermined time has elapsed. When all of the infusion having the powdered drug dissolved therein is to be withdrawn from the drug container, after executing the dissolving operation, the drug-mixing apparatus withdraws the infusion having the powdered drug dissolved therein, from the drug container after a second predetermined time shorter than the first predetermined time has elapsed.

According to a fourth aspect of the present invention, there is provided a drug-mixing apparatus, including: a housing unit into which a syringe used in a drug-mixing operation of mixing a drug housed in a drug container into an infusion housed in an infusion container is allowed to be discarded; and a holding unit, which is configured to hold the syringe under a state of being in abutment against a flange of the syringe, and to discard the syringe into the housing unit by releasing a state of the holding.

According to the first aspect of the present invention, a user can be released from the dangerous work of removing the needle from the discarded syringe.

Further, according to the second aspect and the third aspect of the present invention, the time required for the drug-mixing operation can be shortened.

### BRIEF DESCRIPTION OF THE DRAWINGS

FIG. 1 is a perspective view for illustrating an example of an overall configuration of a drug-mixing apparatus.
FIG. 2 is a perspective view for illustrating an example of a schematic internal configuration of the drug-mixing apparatus when viewed from the front.
FIG. 3 is a perspective view for illustrating an example of a schematic internal configuration of the drug-mixing apparatus when viewed from the rear.
FIG. 4 is a block diagram for illustrating an example of an overall configuration of the drug-mixing apparatus.
FIG. 5 is a perspective view for illustrating an example of an overall configuration of syringe shelves and vial shelves.
FIG. 6 is a view for illustrating an example of a configuration of one syringe shelf and one vial shelf when viewed from a first conveyance unit side.
FIG. 7 shows a perspective view and a bottom view for illustrating an example of each of a centric-tip syringe and an eccentric-tip syringe.
FIG. 8 is a view for illustrating a modification example of an equipment holding unit.
FIG. 9 is a perspective view for illustrating an example of an external appearance of a needle removal unit.
FIG. 10 is a sectional view for illustrating an example of a cross-section of the needle removal unit perpendicular to an insertion direction of a needle attached to a syringe.
FIG. 11 is a schematic perspective view for illustrating an example of a positional relationship between the needle removal unit and a waste box.
FIG. 12 is a view for illustrating a modification example of the needle removal unit.
FIG. 13 is a schematic view for illustrating an example of a positional relationship between a barrel tip of a syringe held by an equipment holding unit and a barrel tip detection unit.
FIG. 14 is a view for illustrating an example of a configuration of a first conveyance unit.
FIG. 15 is a view for illustrating an example of a configuration of a second conveyance unit.
FIG. 16 is a perspective view for illustrating a modification example of the second conveyance unit.
FIG. 17 is a perspective view for illustrating a part of a printing/inspection unit.
FIG. 18 is a perspective view for illustrating an example of a configuration of a drug-mixing unit.
FIG. 19 is a front view for illustrating an example of a schematic configuration of the drug-mixing unit.
FIG. 20 is a flowchart for illustrating an example of a process flow at the time of mixing a powdered drug housed in a vial into an infusion.
FIG. 21 is a schematic view for illustrating an operation example of the drug-mixing unit at the time of dissolving a powdered drug in an infusion.
FIG. 22 is a schematic view for illustrating an example of a flow at the time of withdrawing an infusion having a powdered drug dissolved therein, from a vial.
FIG. 23 is a schematic view for illustrating an outline of an operation of the first conveyance unit as a vibration unit.
FIG. 24 is a schematic view for illustrating an outline of an operation in which the first conveyance unit discards a syringe into the waste box.
FIG. 25 is a plan view for illustrating an example of a partition member provided on an upper side of the waste box.
FIG. 26 is a schematic view for illustrating an outline of an operation of pressing a needle cap of a syringe by the first conveyance unit.

### DESCRIPTION OF THE EMBODIMENTS

Now, at least one embodiment of the present invention is described in detail. In each drawing, an X-axis and a Y-axis are axes in two directions perpendicular to each other in a horizontal plane (ground-contacting surface of a drug-mixing apparatus 1). A positive X-axis direction may be referred to as "rightward direction," and a negative X-axis direction may be referred to as "leftward direction." A positive Y-axis direction may be referred to as "deep direction" or "rearward direction," and a negative Y-axis direction may be referred to as "near direction" or "forward direction." A Z-axis is an axis extending vertically to an XY plane, and a positive Z-axis direction may be referred to as "upward direction," and a negative Z-axis direction may be referred to as "downward direction."

### [Overall Configuration of Drug-mixing Apparatus 1]

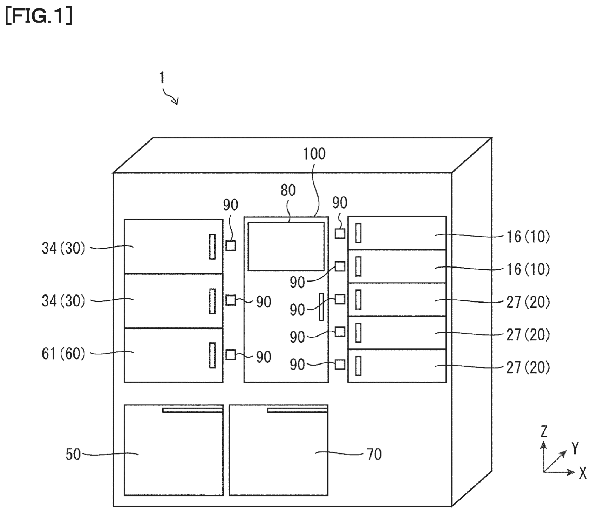

FIG. 1 is a perspective view for illustrating an example of an overall configuration of the drug-mixing apparatus 1. As illustrated in FIG. 1, the drug-mixing apparatus 1 in the at least one embodiment includes syringe shelves 10, vial shelves 20, infusion shelves 30, a printing/inspection unit 50, an infusion receiving shelf 60, a waste box 70, a touch panel 80, and buttons 90.

The drug-mixing apparatus 1 is an apparatus that executes drug-mixing processing (drug-mixing operation) of mixing a drug and an infusion through use of the drug, a syringe, and the infusion indicated in data related to preparation and administration (hereinafter referred to as "preparation/administration data"). The infusion used for the drug-mixing operation may be, for example, a liquid containing saline (normal saline) or glucose.

For example, when the drug indicated in the preparation/administration data is a drug in a liquid state (liquid drug), the drug-mixing apparatus 1 uses a syringe to aspirate the drug from a vial (an example of a drug container) in which the drug has been housed, and injects the drug from the syringe into an infusion container (infusion bag). When the drug indicated in the preparation/administration data is a drug in a solid state (powdered drug), the drug-mixing apparatus 1 uses a syringe to aspirate an infusion from the infusion container, and injects the infusion into the vial. Accordingly, in the vial, the solid drug can be brought into a liquid state. Then, the drug-mixing apparatus 1 uses a syringe to inject the drug in a liquid state (infusion having the drug dissolved therein) into the infusion container. The drug-mixing apparatus 1 executes the drug-mixing operation as described above. The drug-mixing operation may include an operation of aspirating a drug from a vial with use of a syringe, and injecting the drug into another vial. Those drug-mixing operations are executed by a drug-mixing unit 40 described later.

In the drug-mixing apparatus 1, for example, a user can fill the inside of the drug-mixing apparatus 1 with a syringe, a vial, and an infusion container by opening and closing a door. An air cleaning unit described later keeps the inside of the drug-mixing apparatus 1 in a clean state to reduce the possibility that the drug and the infusion are contaminated during the drug-mixing processing. In addition, in order to keep the inside of the drug-mixing apparatus 1 in a clean state, the inside of the drug-mixing apparatus 1 is kept at a positive pressure.

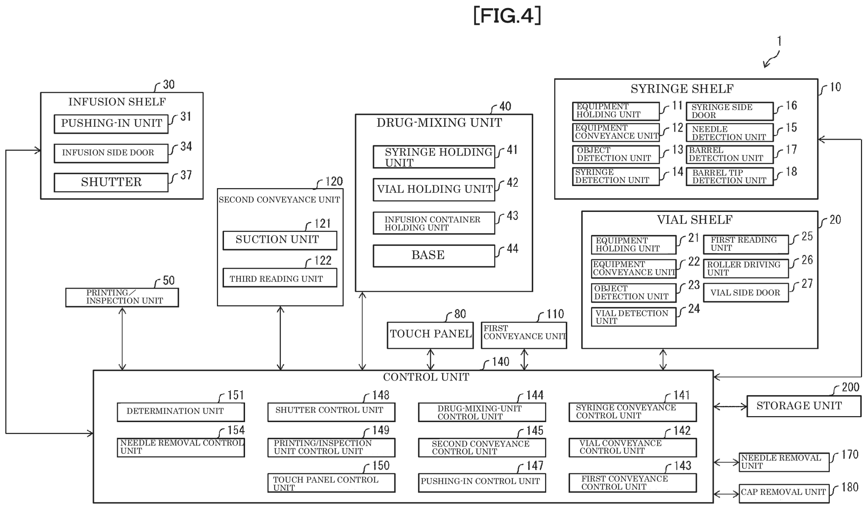

Further, the preparation/administration data is data required for a control unit 140 (see FIG. 4) of the drug-mixing apparatus 1 to perform the drug-mixing processing. At least some pieces of the preparation/administration data may be generated based on prescription data. At least some pieces of the preparation/administration data may be prescription data.

The preparation/administration data includes, for example, information indicating a type of a drug used in the drug-mixing, a prescribed amount of the drug, information indicating a type of a syringe, information indicating a type of an infusion (infusion type information), and information indicating an amount of the infusion to be withdrawn from the infusion container. Further, the preparation/administration data may include information indicating the type (for example, size such as capacity) of the infusion container. The information indicating the type of drug may include information indicating whether the drug is a powdered drug or a liquid drug. The information indicating the type of syringe may include information indicating whether the syringe is a centric-tip syringe or an eccentric-tip syringe, and information indicating the size of the syringe.

Furthermore, the preparation/administration data includes, for example, information relating to the infusion container into which the drug has been injected. Examples of the information include prescription attribute information such as the name of a patient to whom the drug has been prescribed, prescription detail information such as usage and dosage, an order number indicating the order of acceptance of the prescription, and infusion type information (for example, infusion name).

The preparation/administration data described above is merely an example, and among pieces of information described later, information used for drug-mixing may be included in the preparation/administration data.

The syringe shelves 10 are equipment storage shelves capable of storing syringes supplied to the drug-mixing apparatus 1. Each syringe shelf 10 is provided with a syringe side door 16. The user can cause the syringe shelf 10 to hold a syringe by opening and closing the syringe side door 16.

The vial shelves 20 are equipment storage shelves capable of storing vials supplied to the drug-mixing apparatus 1. Each vial shelf 20 is provided with a vial side door 27. The user can cause the vial shelf 20 to hold a vial by opening and closing the vial side door 27.

The infusion shelves 30 are infusion storage shelves capable of storing infusion containers supplied to the drug-mixing apparatus 1. Each infusion shelf 30 is provided with an infusion side door 34. The user can cause the infusion side door 34 to hold an infusion container by opening and closing the infusion side door 34.

The syringe side door 16, the vial side door 27, and the infusion side door 34 are doors that allow user's access to the syringe shelf 10, the vial shelf 20, or the infusion shelf 30 in an open state, and can block the access in a closed state. In the drug-mixing apparatus 1, only one of the syringe side door 16, the vial side door 27, and the infusion side door 34 is controlled by the control unit 140, which is described later, to be in an unlocked state.

The printing/inspection unit 50 is a unit that prints a second label and attaches the second label to an infusion container before or after drug injection. The printing/inspection unit 50 may print an unsuitability information label and attach the unsuitability information label to the infusion container after drug injection. Further, the printing/inspection unit 50 is also a unit that weighs the infusion container before and after drug injection.

A first label on which, for example, the type of infusion and/or the type of infusion container is printed is attached in advance to the infusion container by a pharmaceutical manufacturer that provides the infusion container. The first label is also referred to as "infusion label." Meanwhile, the above-mentioned information relating to the infusion container into which the drug has been injected is information given to the infusion container that is the target of drug injection, and is different from the information included in the first label attached in advance to the infusion container. The printing/inspection unit 50 prints the content of this information on a label and attaches the label to the infusion container as a second label different from the first label.

The unsuitability information label is a label on which unsuitability information is printed. The unsuitability information label may be, for example, a label on which an X mark is printed. The unsuitability information may be, for example, information indicating that the weight measured by the printing/inspection unit 50 is unsuitable, and/or information indicating that the type of drug indicated in the preparation/administration data is not included in the information read from the second label. The unsuitability of the weight means, for example, that the injection amount (prescribed amount) of the drug injected into the infusion container, or the withdrawal amount of the infusion withdrawn from the infusion container does not correspond to the amount indicated in the preparation/administration data. The control unit 140 described later determines whether the weight is suitable, and whether the type of drug indicated in the preparation/administration data is included in the information read from the second label.

The infusion receiving shelf 60 is a unit that receives the infusion container after drug injection to which the second label is attached. The infusion receiving shelf 60 is provided with an infusion receiving door 61. The waste box 70 is a box that receives used syringes and vials (syringes and vials used in the drug-mixing operation). Further, the waste box 70 is also a box that receives a needle cap removed from a syringe 501. The touch panel 80 has a function of an operation unit that receives various types of operations by the user and a function of a display unit that displays various types of information. The operation unit and the display unit may be provided as separate members. Further, instead of the display unit, a presentation unit (e.g., a speaker) that presents various types of information may be provided.

The buttons 90 are mechanical buttons capable of receiving a user operation in order to unlock each of the syringe side door 16, the vial side door 27, the infusion side door 34, and the infusion receiving door 61. Corresponding one of the buttons is provided to each of the syringe side door 16, the vial side door 27, the infusion side door 34, and the infusion receiving door 61. When being brought into a closed state, the syringe side door 16, the vial side door 27, the infusion side door 34, and the infusion receiving door 61 are locked by a locking mechanism (not shown). The syringe side door 16, the vial side door 27, the infusion side door 34, and the infusion receiving door 61 are unlocked only when a user presses the buttons 90. However, in a case in which any of the doors is unlocked to be openable, even when the control unit 140 described later receives a user operation on the button 90 corresponding to a door that is in a closed and locked state, the control unit 140 invalidates the user operation. Accordingly, the control unit 140 maintains the doors other than the unlocked door, in a closed and locked state.

Even when the button 90 corresponding to the door of the syringe shelf 10, the vial shelf 20, the infusion shelf 30, or the infusion receiving shelf 60, to which a first conveyance unit 110 or a second conveyance unit 120 described later is accessing is pressed, the control unit 140 does not unlock the door.

Further, a light-emitting member (not shown) (for example, a light emitting diode (LED)) may be provided to each of the syringe shelf 10, the vial shelf 20, the infusion shelf 30, and the infusion receiving shelf 60 so as to correspond to each of those shelves. The control unit 140 may light up the light-emitting member corresponding to the shelf to which the first conveyance unit 110 or the second conveyance unit 120 accesses. In this case, a locking mechanism for each door is not always required to be provided.

The drug-mixing apparatus 1 is further provided with a central door 100. The central door 100 is a door that allows user access to each unit provided inside the drug-mixing apparatus 1 in an open state, and can block the access in a closed state. Examples of the respective units include the drug-mixing unit 40 and the first conveyance unit 110. The central door 100 is in a locked state when the drug-mixing apparatus 1 is under operation.

### [Outline of Internal Configuration of Drug-mixing apparatus]

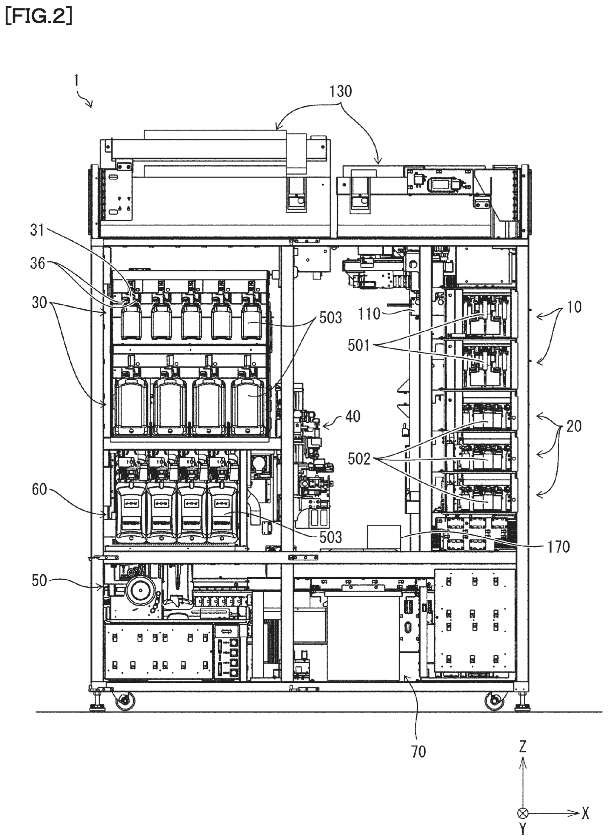

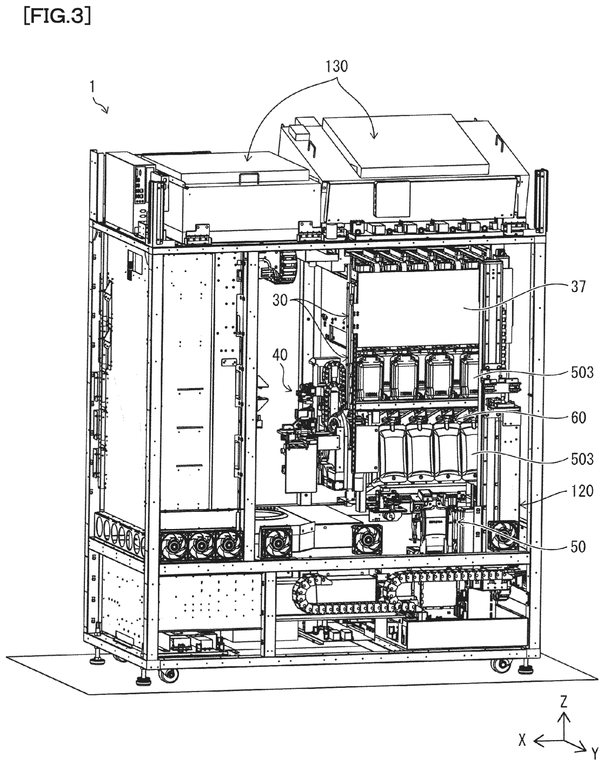

FIG. 2 is a perspective view for illustrating an example of a schematic internal configuration of the drug-mixing apparatus 1 when viewed from the front (front side). FIG. 3 is a perspective view for illustrating an example of a schematic internal configuration of the drug-mixing apparatus 1 when viewed from the rear (deep direction).

As illustrated in FIG. 2 and FIG. 3, the drug-mixing apparatus 1 includes, in addition to the above-mentioned syringe shelves 10, vial shelves 20, infusion shelves 30, printing/inspection unit 50, infusion receiving shelf 60, and waste box 70, the drug-mixing unit 40, the first conveyance unit 110, the second conveyance unit 120, air cleaning units 130, and a needle removal unit 170. Further, the drug-mixing apparatus 1 includes a cap removal unit 180 (see FIG. 4), although not illustrated in FIG. 2 and FIG. 3.

The drug-mixing unit 40 functions as a drug-mixing unit that mixes a drug (powdered drug or liquid drug) housed in a vial 502 into an infusion housed in an infusion container 503. In the at least one embodiment, the drug-mixing unit 40 is located between the syringe shelf 10 and the vial shelf 20, and the infusion shelf 30 and the infusion receiving shelf 60.

The drug-mixing unit 40 executes a drug-mixing operation based on the preparation/administration data. When the prescription data forms a prescription data group including a plurality of pieces of prescription data, the drug-mixing operation is executed based on the prescription data group. The drug-mixing unit 40 uses a syringe 501 to execute a drug-mixing operation of mixing a drug housed in the vial 502 into an infusion housed in the infusion container 503. When the drug housed in the vial 502 is a powdered drug, the drug-mixing unit 40 dissolves the powdered drug housed in the vial 502, with an infusion, and uses the syringe 501 to execute a drug-mixing operation of mixing the infusion having the powdered drug dissolved therein, into the infusion housed in the infusion container 503.

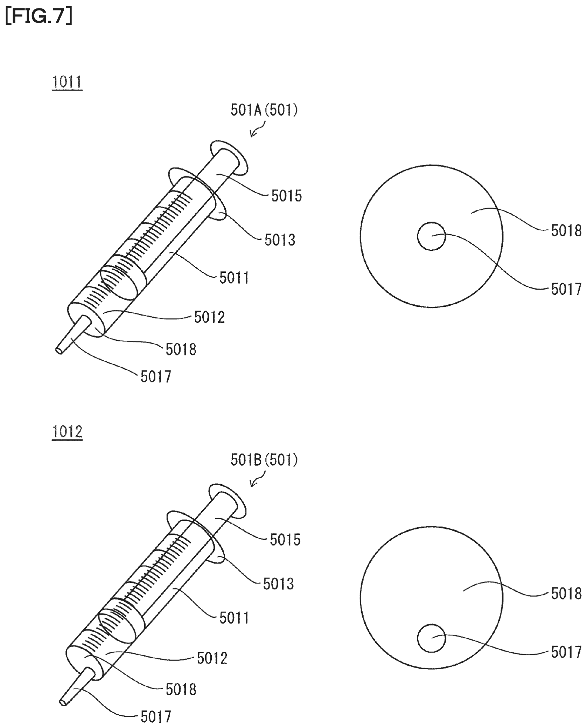

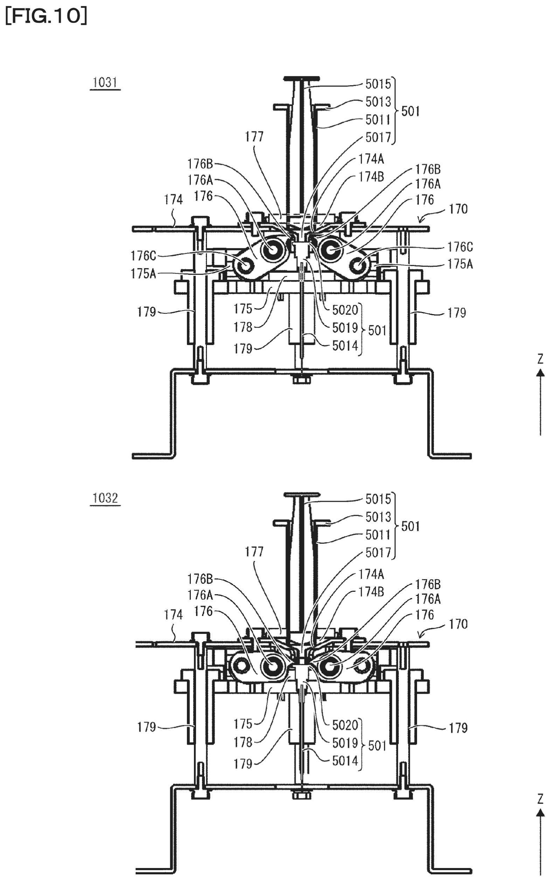

As illustrated in FIG. 10 described later, the syringe 501 includes a barrel (syringe barrel) 5011, a needle 5014, and a plunger 5015. The plunger 5015 is a pusher that moves in and out of the barrel 5011. With the plunger 5015 moving in and out of the barrel 5011, a drug or the like can be drawn into the barrel 5011, or the drug or the like in the barrel 5011 can be discharged. The barrel 5011 includes: a needle-side end portion 5012 (see FIG. 7) provided with a barrel tip 5017; and a flange 5013 that is an end portion on a side opposite to the needle-side end portion 5012, and on a side from which the plunger 5015 is allowed to move in and out of the barrel 5011.

The needle 5014 is attached to the barrel tip 5017. Specifically, an insertion hub 5019 is attached to the barrel tip 5017, and the needle 5014 is attached to the insertion hub 5019 so that the needle 5014 is attached to the syringe 501 via the insertion hub 5019. The insertion hub 5019 includes a flange 5020 that is an end portion on a side to be attached to the barrel tip 5017.

The first conveyance unit 110 conveys a syringe 501 indicated in preparation/administration data from the syringe shelf 10 to the drug-mixing unit 40, or conveys a vial 502 in which a drug indicated in the preparation/administration data is housed from the vial shelf 20 to the drug-mixing unit 40. When the preparation/administration data is input, the first conveyance unit 110 conveys the syringe 501 stored in the syringe shelf 10 and the vial 502 stored in the vial shelf 20 to the drug-mixing unit 40. In the at least one embodiment, the first conveyance unit 110 grips an upper part of a barrel portion of the syringe 501 stored in the syringe shelf 10, and delivers the syringe 501 to the drug-mixing unit 40. Similarly, the first conveyance unit 110 grips a neck portion of the vial 502 stored in the vial shelf 20, and delivers the vial 502 to the drug-mixing unit 40.

A needle cap has been attached to the needle 5014 of the syringe 501 supplied into the syringe shelf 10. Accordingly, the first conveyance unit 110 conveys the syringe 501 stored in the syringe shelf 10, to the cap removal unit 180 before conveying the syringe 501 to the drug-mixing unit 40. As a result, the first conveyance unit 110 can remove the needle cap attached to the needle 5014 of the syringe 501, and can convey the syringe 501 from which the needle cap has been removed, to the drug-mixing unit 40. Further, the first conveyance unit 110 conveys and discards the needle cap removed by the cap removal unit 180, to the waste box 70.

The first conveyance unit 110 conveys the syringe 501 used in the drug-mixing operation (unrequired syringe 501) to the needle removal unit 170. Then, the first conveyance unit 110 conveys the syringe 501 from which the needle 5014 has been removed by the needle removal unit 170, to the waste box 70. Further, the first conveyance unit 110 conveys the vial 502 used in the drug-mixing operation (unrequired vial 502) to the waste box 70. However, depending on the type of the vial 502, the first conveyance unit 110 is not always required to convey the vial 502 to the waste box 70.

The first conveyance unit 110 functions as a first equipment removal unit that removes the syringe 501 and the vial 502 held by the drug-mixing unit 40, from the drug-mixing unit 40. The first equipment removal unit is an example of an equipment removal unit. The first conveyance unit 110 removes each of the syringe 501 and the vial 502 from the drug-mixing unit 40, for example, after the drug-mixing operation by the drug-mixing unit 40 is completed. Alternatively, the first conveyance unit 110 removes each of the syringe 501 and the vial 502 from the drug-mixing unit 40, for example, for temporary holding during the drug-mixing operation by the drug-mixing unit 40.

The first conveyance unit 110 is a common conveyance unit for the syringe 501 and the vial 502, which conveys the syringe 501 and the vial 502, but the configuration is not limited thereto. A dedicated conveyance unit that conveys the syringe 501 and a dedicated conveyance unit that conveys the vial 502 may be provided, respectively.

The second conveyance unit 120 conveys the infusion container 503 indicated in the preparation/administration data among the infusion shelf 30, the drug-mixing unit 40, the printing/inspection unit 50, and the infusion receiving shelf 60.

The second conveyance unit 120 conveys the infusion container 503 to the drug-mixing unit 40. When the preparation/administration data is input, the second conveyance unit 120 conveys the infusion container 503 stored in the infusion shelf 30 to the drug-mixing unit 40. In the at least one embodiment, the second conveyance unit 120 suctions a body of the infusion container 503 stored in the infusion shelf 30 to deliver the infusion container 503 to the drug-mixing unit 40.

In addition, the second conveyance unit 120 conveys the infusion container 503 between the drug-mixing unit 40 and the printing/inspection unit 50. In the at least one embodiment, for example, the second conveyance unit 120 suctions the body of the infusion container 503 after drug injection to convey the infusion container 503 to the printing/inspection unit 50. Further, the second conveyance unit 120 delivers the infusion container 503 to which the second label or the unsuitability information label has been attached by the printing/inspection unit 50, to the infusion receiving shelf 60.

The second conveyance unit 120 functions as a second equipment removal unit that removes the infusion container 503 held by the drug-mixing unit 40, from the drug-mixing unit 40. The second equipment removal unit is an example of an equipment removal unit. The second conveyance unit 120 removes the infusion container 503 from the drug-mixing unit 40, for example, after the drug-mixing operation by the drug-mixing unit 40 is completed. The second conveyance unit 120 may remove the infusion container 503 from the drug-mixing unit 40 during the drug-mixing operation by the drug-mixing unit 40, in order to weigh the infusion container 503 given after withdrawing the infusion for dissolving the powdered drug, in the printing/inspection unit 50.

In the at least one embodiment, the first conveyance unit 110 and the second conveyance unit 120 are separate conveyance units that operate independently, but a common conveyance unit for the syringe 501, the vial 502, and the infusion container 503, which conveys the syringe 501, the vial 502, and the infusion container 503, may be used. In this case, the common conveyance unit may function as an equipment removal unit that removes the syringe 501, the vial 502, and the infusion container 503 held by the drug-mixing unit 40, from the drug-mixing unit 40.

Each of the air cleaning units 130 cleans the air inside the drug-mixing apparatus 1 and exhausts the air. In the at least one embodiment, the air cleaning units 130 are provided in an upper part of the drug-mixing apparatus 1. The air cleaned by the air cleaning units 130 flows from the upper part to a bottom part of the drug-mixing apparatus 1. Each of the air cleaning units 130 includes, for example, a high-efficiency particulate air (HEPA) filter.

The needle removal unit 170 is a needle removal part that removes the needle 5014 from the syringe 501 having the needle 5014 attached thereto. The phrase "removing the needle 5014 from the syringe 501" means removing the needle 5014 from the barrel 5011 of the syringe 501. The needle removal unit 170 removes the needle 5014 having no needle cap attached thereto, from the syringe 501.

In the at least one embodiment, the needle removal unit 170 removes the needle 5014 from the syringe 501 used in the drug-mixing operation, after the drug-mixing operation by the drug-mixing unit 40 is completed. In the at least one embodiment, after the first conveyance unit 110 inserts the needle 5014 attached to the syringe 501 used in the drug-mixing operation, into the needle removal unit 170, the needle removal unit 170 removes the needle 5014 from the syringe 501. When drug-mixing operations using the same type of drug are performed consecutively, the needle removal unit 170 removes the needle 5014 attached to the syringe 501 used in the drug-mixing operation, after the last drug-mixing operation among the consecutive drug-mixing operations is completed. In this case, the drug-mixing apparatus 1 can reuse the same syringe 501.

The needle removal unit 170 removes the needle 5014 from the syringe 501 used in the drug-mixing operation, and hence it is not required that a user perform the work of removing the needle 5014 from the syringe 501. Accordingly, the user can be released from the dangerous work of removing the needle 5014 from the syringe 501.

The cap removal unit 180 is a member that removes the needle cap of the syringe 501. The cap removal unit 180 removes the needle cap attached to the needle 5014 of the syringe 501 before being conveyed to the drug-mixing unit 40, and holds the needle cap. The cap removal unit 180 removes the needle cap from the syringe 501 by holding the needle cap attached to the needle 5014 of the syringe 501 when the first conveyance unit 110 inserts the syringe 501 into the cap removal unit 180. The cap removal unit 180 removes only the needle cap without removing the needle 5014 from the syringe 501.

Further, as illustrated in FIG. 2, the infusion shelf 30 includes a pushing-in unit 31 and rail portions 36. Further, as illustrated in FIG. 3, the infusion shelf 30 includes a shutter 37.

The rail portions 36 are each a member that is provided inside the infusion shelf 30 and extends in a deep direction of the drug-mixing apparatus 1 (that is, in the +Y-axis direction) from the infusion side door 34 (see FIG. 1) side. In each of the rail portions 36, an end portion located on the infusion side door 34 side may be also referred to as "door-side end portion," and an end portion located on a deep side of the drug-mixing apparatus 1 may be also referred to as "deep-side end portion." The infusion container 503 is loaded onto the rail portions 36 from the door-side end portion, and is taken out by the second conveyance unit 120 from the deep-side end portion. At least one infusion container 503 can be suspended from the rail portions 36.

In the at least one embodiment, each infusion shelf 30 is provided with a plurality of pairs of rail portions 36. However, the number of pairs of rail portions 36 provided in each infusion shelf 30 is not limited. Further, in the at least one embodiment, the same type of infusion container 503 (infusion containers 503, into each of which the same type of infusion is housed, and which have the same size) is loaded onto each of the pair of rail portions 36. Then, for each infusion shelf 30, the infusion container 503 to be supplied is determined. However, different types of infusion containers 503 may be loaded onto the pairs of rail portions 36, respectively.

The pushing-in unit 31 is a member that is movable along the rail portions 36 and pushes the infusion container 503 suspended from the rail portions 36, from the door-side end portion to the deep-side end portion. In the at least one embodiment, the pushing-in unit 31 is connected to a pushing-in conveyance unit. The pushing-in conveyance unit is provided along the rail portions 36. The pushing-in conveyance unit can move the pushing-in unit 31 along the rail portions 36 by operating under the control of a pushing-in control unit 147. The pushing-in conveyance unit may be, for example, an endless rotating member. The size of the pushing-in unit 31 and the positional relationship between the rail portions 36 and the pushing-in conveyance unit 35 are defined so that the infusion container 503 suspended from the rail portions 36 can be pushed in by the movement of the pushing-in unit 31.

The pushing-in unit 31 pushes the infusion container 503 suspended from the rail portions 36, from the door-side end portion to the deep-side end portion, triggered by the infusion side door 34 being brought into a locked state. In the at least one embodiment, when the infusion side door 34 is brought into a closed state and then is locked, the pushing-in control unit 147 controls the pushing-in conveyance unit to move the pushing-in unit 31 from the door-side end portion to the deep-side end portion. A detection unit that detects the infusion container 503 present at the deep-side end portion is provided at the deep-side end portion of a wall portion forming the infusion shelf 30. The pushing-in control unit 147 moves the pushing-in unit 31 toward the deep-side end portion side until the detection unit detects the infusion container 503.

Further, consideration is made on a case in which the second conveyance unit 120 takes out the infusion container 503 located at the deep-side end portion under a state in which a plurality of infusion containers 503 are suspended from the rail portions 36. In this case, after the second conveyance unit 120 takes out the infusion container 503, the pushing-in unit 31 pushes the remaining infusion containers 503 suspended from the rail portions 36, from the door-side end portion to the deep-side end portion. In the at least one embodiment, when the second conveyance unit 120 has taken out the infusion container 503 located at the deep-side end portion, the pushing-in control unit 147 controls the pushing-in conveyance unit to further move the pushing-in unit 31 toward the deep-side end portion.

The shutter 37 is located on the deep side of the infusion shelf 30 in which the infusion side door 34 is open, and blocks the deep-side end portion from a region on the deep direction (+Y-axis direction) side thereof so that a user does not touch the second conveyance unit 120 even when the user puts his or her hand into the infusion shelf 30 from the infusion side door 34 side. The shutter 37 is controlled to be movable in an up-down direction (±Z-axis direction) by a shutter control unit 148.

The shutter 37 is controlled by the shutter control unit 148 to be located on the deep side of the infusion shelf 30 corresponding to the infusion side door 34, for example, when the infusion side door 34 is opened. For example, when the shutter control unit 148 receives a user operation on the button 90, the shutter control unit 148 moves the shutter 37 to a position facing the deep-side end portion of the infusion shelf 30 corresponding to the button 90.

Further, when the second conveyance unit 120 takes out the infusion container 503, the shutter 37 moves to an infusion shelf 30 other than the infusion shelf 30 in which the infusion container 503 is stored. For example, when the shutter 37 is located at a position facing the infusion shelf 30 in which the infusion container 503 to be taken out by the second conveyance unit 120 is held, the shutter control unit 148 moves the shutter 37 to a position facing another infusion shelf 30. Information indicating the storage position of the infusion container 503 in the infusion shelf 30 is stored in a storage unit 200 in association with, for example, infusion type information.

The arrangement mode of each member in the drug-mixing apparatus 1 is not limited to the above-mentioned arrangement mode. Further, in the at least one embodiment, the syringe shelf 10, the vial shelf 20, and the infusion shelf 30 are each provided in a plurality of stages, but may each be provided in one stage. In the at least one embodiment, the infusion receiving shelf 60 is provided in one stage, but may be provided in a plurality of stages. Further, the syringe side door 16 and/or the vial side door 27 may be provided on a side surface (right side on the drawing sheet of FIG. 2) of the drug-mixing apparatus 1. In this case, a plurality of syringes 501 or a plurality of vials 502 can be supplied from the side surface side of the drug-mixing apparatus 1. When the infusion shelf 30 has the same configuration as in the syringe shelf 10 and the vial shelf 20, the infusion side door 34 may be provided on a side surface side (left side on the drawing sheet of FIG. 2) of the drug-mixing apparatus 1.

### [Other Configurations]

FIG. 4 is a block diagram for illustrating an example of an overall configuration of the drug-mixing apparatus 1. As illustrated in FIG. 4, the drug-mixing apparatus 1 includes the control unit 140 and the storage unit 200. In FIG. 4, illustration is given of only the main hardware configurations that can be controlled by the control unit 140, among the hardware configurations included in the drug-mixing apparatus 1.

The control unit 140 comprehensively controls the operation of the drug-mixing apparatus 1 based on the preparation/administration data. The control unit 140 includes, for example, a syringe conveyance control unit 141, a vial conveyance control unit 142, a first conveyance control unit 143, a drug-mixing-unit control unit 144, and a second conveyance control unit 145. The control unit 140 includes, for example, the pushing-in control unit 147, the shutter control unit 148, a printing/inspection unit control unit 149, a touch panel control unit 150, a determination unit 151, and a needle removal control unit 154.

The syringe conveyance control unit 141 controls operations of members related to the conveyance of the syringe 501, in the syringe shelf 10. The vial conveyance control unit 142 controls operations of members related to the conveyance of the vial 502, in the vial shelf 20. The drug-mixing-unit control unit 144 controls operations of members included in the drug-mixing unit 40.

The first conveyance control unit 143 controls operations of members included in the first conveyance unit 110. The first conveyance control unit 143 controls, for example, movement of the first conveyance unit 110 among the syringe shelf 10, the vial shelf 20, the drug-mixing unit 40, the waste box 70, the needle removal unit 170, and the cap removal unit 180. The second conveyance control unit 145 controls operations of members included in the second conveyance unit 120. The second conveyance control unit 145 controls, for example, movement of the second conveyance unit 120 among the infusion shelf 30, the drug-mixing unit 40, the printing/inspection unit 50, and the infusion receiving shelf 60.

The pushing-in control unit 147 controls operations of members related to the conveyance of the infusion container 503, in the infusion shelf 30. The shutter control unit 148 controls movement of the shutter 37 (see FIG. 3). The printing/inspection unit control unit 149 controls operations of members included in the printing/inspection unit 50. The touch panel control unit 150 controls the touch panel 80. The needle removal control unit 154 controls operations of members included in the needle removal unit 170.

Further, data for the control unit 140 to perform various types of processing (for example, drug-mixing processing) is stored in the storage unit 200. For example, preparation/administration data is stored in the storage unit 200. The information stored in the storage unit 200 may be stored in a storage device external to the drug-mixing apparatus 1.

### [Specific Configuration of Syringe Shelf]

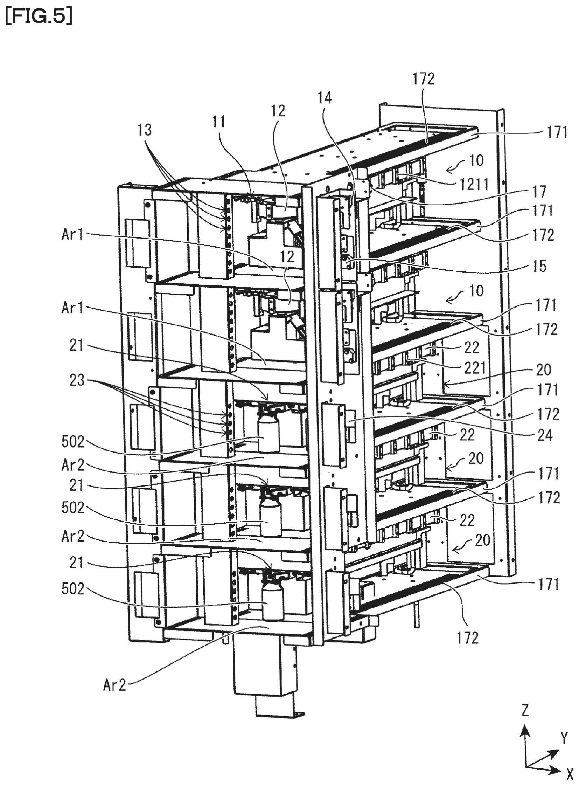

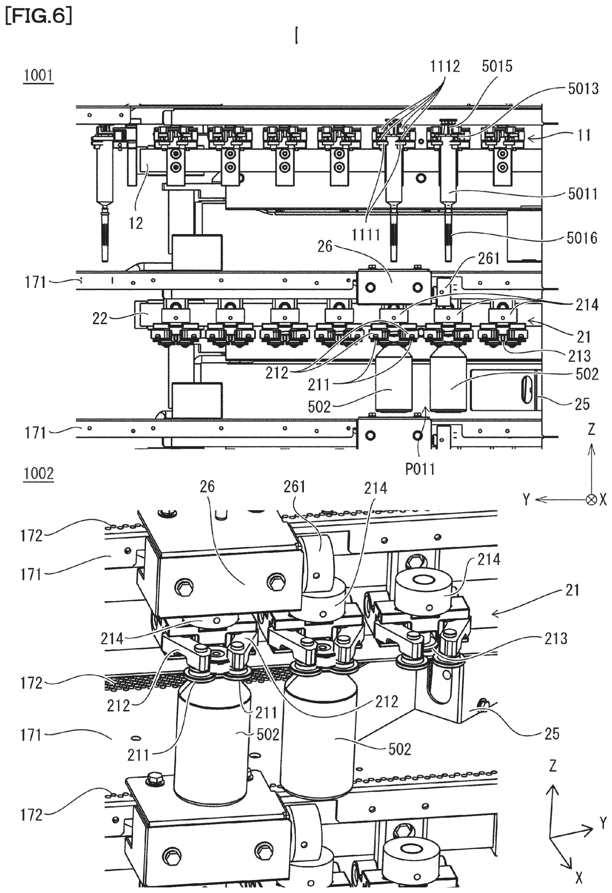

FIG. 5 is a perspective view for illustrating an example of an overall configuration of the syringe shelves 10 and the vial shelves 20. FIG. 6 is a view for illustrating an example of a configuration of one syringe shelf 10 and one vial shelf 20 when viewed from the first conveyance unit 110 side.

As illustrated in FIG. 5, the syringe shelf 10 includes a plurality of equipment holding units 11, an equipment conveyance unit 12, object detection units 13, a syringe detection unit 14, a needle detection unit 15, a barrel detection unit 17. Although an equipment holding unit 11 is also attached to an attachment portion 1211 of the equipment conveyance unit 12 illustrated in FIG. 5, illustration thereof is omitted.

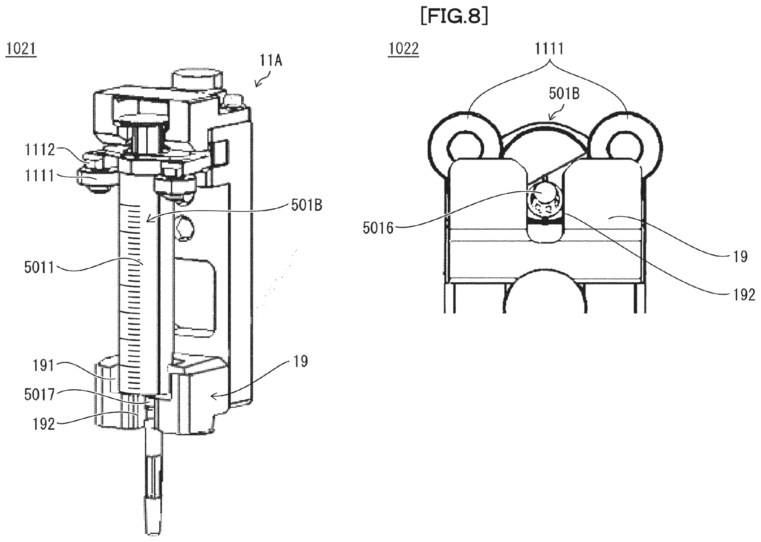

Prior to drug-mixing processing, the syringe shelf 10 is filled with the syringes 501 by the user. The syringe 501 is supplied to the syringe shelf 10 with the needle cap 5016 (see FIG. 8) attached to the needle 5014.

In the at least one embodiment, a plurality of holes 172 are formed in a plate-like member 171 that defines each syringe shelf 10. As a result, for example, the air cleaned by the air cleaning units 130 (see FIG. 2) can efficiently flow from the upper part to the bottom part of the drug-mixing apparatus 1.

The plurality of equipment holding units 11 are members (holding units) capable of holding the syringes 501 supplied into the drug-mixing apparatus 1. In the at least one embodiment, one syringe 501 is held in one equipment holding unit 11, but a plurality of syringes 501 may be held in one equipment holding unit 11. In the at least one embodiment, as illustrated in FIG. 6, the equipment holding unit 11 includes free rollers 1111 and equipment clamping units 1112. In the specification, a member described as clamping an object is merely an example, and the member may be any member that can hold the object.

The equipment clamping units 1112 clamp the syringe 501. In the at least one embodiment, the equipment clamping units 1112 clamp the flange 5013 of the syringe 501. The equipment clamping units 1112 are provided at such a height that the syringe 501 does not touch the plate-like member 171 when the syringe 501 is held by the equipment holding unit 11. A pair of equipment clamping units 1112 are biased in a direction toward each other so as to be able to clamp the syringe 501.

Below a tip portion of each of the equipment clamping units 1112, the free roller 1111 which has a rotation axis extending in the up-down direction and is rotatable on the XY plane is provided. The free roller 1111 holds a vicinity of the flange 5013 of the barrel 5011 clamped by the equipment clamping units 1112.

The equipment conveyance unit 12 is a member that conveys at least one of the plurality of equipment holding units 11 to a work area Ar1. In the at least one embodiment, the equipment conveyance unit 12 is an endless rotating member (rotating belt) to which the plurality of equipment holding units 11 are connected. Accordingly, in the at least one embodiment, the equipment conveyance unit 12 conveys the equipment holding units 11 one by one to the work area Ar1. However, the equipment conveyance unit 12 may be a conveyance unit that conveys the plurality of equipment holding units 11 to the work area Ar1 at once.

The work area Ar1 is an area in which the user causes at least one of the plurality of equipment holding units 11 to hold the syringe 501. The work area Ar1 is also an area in which the user removes the syringe 501 held by at least one of the plurality of equipment holding units 11. In the at least one embodiment, the work area Ar1 is an area in which the user causes one equipment holding unit 11 to hold the syringe 501 and also an area in which the user removes the syringe 501 held by one equipment holding unit 11.

The work area Ar1 may function only as an area in which the user causes at least one of the plurality of equipment holding units 11 to hold the syringe 501. In this case, an area in which the user removes the syringe 501 held by the equipment holding unit 11 may be provided at a position different from the work area Ar1. The work area Ar1 may also function only as an area in which the user removes the syringe 501 held by the equipment holding unit 11. In this case, an area in which the user causes the equipment holding unit 11 to hold the syringe 501 may be provided at a position different from the work area Ar1.

In the at least one embodiment, the equipment conveyance unit 12 conveys another equipment holding unit 11 in place of the equipment holding unit 11 located in the work area Ar1, triggered by such detection of a user's action that the object detection units 13 no longer detect an object (e.g., a user's hand) after detecting the object. In this case, when the user's hand is inserted into the work area Ar1, the user causes the equipment holding unit 11 to hold the syringe 501, and then the user's hand is moved away from the work area Ar1, the equipment conveyance unit 12 conveys another equipment holding unit 11 to the work area Ar1. In addition to the detection of the user's action, the equipment conveyance unit 12 may convey another equipment holding unit 11 to the work area Ar1, triggered by the syringe detection unit 14 detecting the syringe 501 held by the equipment holding unit 11, or by the syringe detection unit 14 being unable to detect the syringe 501 held by the equipment holding unit 11.

The equipment conveyance unit 12 may convey another equipment holding unit 11 in place of the equipment holding unit 11 located in the work area Ar1, triggered by a conveyance instruction from the user. In this case, the syringe conveyance control unit 141 may receive the conveyance instruction from the user through, for example, the touch panel 80.

The equipment conveyance unit 12 changes the equipment holding unit 11 located in the work area Ar1 by rotating, for example, counterclockwise. With this conveyance operation, another equipment holding unit 11 in which the syringe 501 is not held can be conveyed to the work area Ar1, in place of the equipment holding unit 11 in which the syringe 501 is held in the work area Ar1.

Each of the object detection units 13 is a member that detects an object inserted into the work area Ar1. In the at least one embodiment, the object detection units 13 detect a user's hand or arm in a state of being inserted into the work area Ar1.

The syringe detection unit 14 is a member that detects the syringe 501 held by the equipment holding unit 11 located in the work area Ar1. In the at least one embodiment, when the syringe 501 is held by the equipment holding unit 11, the syringe detection unit 14 detects the barrel 5011.

The needle detection unit 15 is a detection unit that detects the needle 5014 of the syringe 501 held by the equipment holding unit 11 located in the work area Ar1. In the at least one embodiment, the needle detection unit 15 detects the needle cap 5016 attached to the needle 5014. The needle detection unit 15 may function as a syringe detection unit. In this case, the syringe detection unit 14 is not always required to be provided.

The barrel detection unit 17 is a member capable of detecting the thickness of the barrel 5011. The barrel detection unit 17 is provided at a position at which the barrel detection unit 17 can detect the barrel 5011 being conveyed by the equipment conveyance unit 12. The barrel detection unit 17 continues to detect the barrel 5011 being conveyed, for example.

Further, as illustrated in FIG. 4, the syringe shelf 10 may be provided with a barrel tip detection unit 18 that detects a barrel tip of the barrel 5011 held by the equipment holding unit 11 located in the work area Ar1. The barrel tip detection unit 18 may be provided, for example, at a position below the syringe detection unit 14 so as to be able to detect the barrel tip 5017 of the syringe 501 held by the equipment holding unit 11.

### [Type of Syringe and Modification Example of Equipment Holding Unit]

Reference numeral 1011 in FIG. 7 indicates a perspective view and a bottom view for illustrating an example of a centric-tip syringe 501A in which a barrel tip 5017 of a barrel 5011 is of a centric tip type. Reference numeral 1012 in FIG. 7 indicates a perspective view and a bottom view for illustrating an example of an eccentric-tip syringe 501B in which a barrel tip 5017 of a barrel 5011 is of an eccentric tip type.

The centric-tip syringe 501A is a syringe in which, as indicated by reference numeral 1011 in FIG. 7, the barrel tip 5017 is provided at the center of a bottom part 5018 of the barrel 5011. The eccentric-tip syringe 501B is a syringe in which, as indicated by reference numeral 1012 in FIG. 7, the barrel tip 5017 is provided at a position deviated from the center of the bottom part 5018 of the barrel 5011.

The capacities of the centric-tip syringe 501A and the eccentric-tip syringe 501B (thicknesses; outer diameters of the syringes 501) may be different from each other. For example, the capacity (thickness) of the eccentric-tip syringe 501B may be larger than the capacity (thickness) of the centric-tip syringe 501A.

The syringe shelf 10 may house the centric-tip syringe 501A and the eccentric-tip syringe 501B as the syringes 501. In this case, the drug-mixing apparatus 1 can use the centric-tip syringe 501A and the eccentric-tip syringe 501B for the drug-mixing operation.

When the centric-tip syringe 501A and the eccentric-tip syringe 501B are housed in the syringe shelf 10, the syringe shelf 10 may include an equipment holding unit 11A illustrated in FIG. 8. FIG. 8 is a view for illustrating the equipment holding unit 11A that is a modification example of the equipment holding unit 11. Reference numeral 1021 in FIG. 8 indicates a perspective view of the equipment holding unit 11A, and reference numeral 1022 in FIG. 8 indicates a bottom view of the equipment holding unit 11A. FIG. 8 shows an example in which the eccentric-tip syringe 501B is mounted on the equipment holding unit 11A.

As illustrated in FIG. 8, the equipment holding unit 11A includes a fixing member 19 that fixes the position of the syringe 501 held by the equipment holding unit 11A. The fixing member 19 fixes the eccentric-tip syringe 501B held by the equipment holding unit 11A so that the eccentric-tip syringe 501B does not rotate about the axis of the eccentric-tip syringe 501B.

In the at least one embodiment, the fixing member 19 includes: a first insertion part 191 into which the barrel 5011 is to be inserted; and a second insertion part 192 into which the barrel tip 5017 is to be inserted.

The first insertion part 191 has a shape and a size that allow insertion of the centric-tip syringe 501A and the eccentric-tip syringe 501B. Specifically, the shape and the size of the first insertion part 191 are defined so as to allow the barrel tip 5017 (needle cap 5016) to be inserted into the second insertion part 192 both when the centric-tip syringe 501A is inserted into the first insertion part 191 and when the eccentric-tip syringe 501B is inserted into the first insertion part 191.

The second insertion part 192 has a shape and a size that allow insertion of the barrel tip 5017 of each of the centric-tip syringe 501A and the eccentric-tip syringe 501B inserted into the first insertion part 191, and that can restrict the rotation of the eccentric-tip syringe 501B inserted into the first insertion part 191 about its axis. As indicated by reference numeral 1022 in FIG. 8, the second insertion part 192 may have a groove shape extending in an insertion direction of the syringe 501 to be inserted into the equipment holding unit 11A.

As indicated by reference numeral 1012 in FIG. 7, the scale of the eccentric-tip syringe 501B is provided on a surface of the barrel 5011 on a side opposite to a side on which the barrel tip 5017 is provided, across the central axis of the eccentric-tip syringe 501B. The shape of the second insertion part 192 is defined so as to allow the eccentric-tip syringe 501B to be held by the equipment holding unit 11A such that the scale provided as described above faces forward (front side).

Accordingly, as indicated by reference numeral 1021 in FIG. 8, a user can cause the equipment holding unit 11A to hold the eccentric-tip syringe 501B such that the scale faces forward. Then, with the equipment holding unit 11A being caused to hold the eccentric-tip syringe 501B as described above, the first conveyance unit 110 can cause the drug-mixing unit 40 to hold the eccentric-tip syringe 501B such that the scale faces the front side of the drug-mixing unit 40 (in the +X-axis direction in the at least one embodiment).

### [Specific Configuration of Vial Shelf]

As illustrated in FIG. 5 and indicated by reference numeral 1001 in FIG. 6, the vial shelf 20 includes a plurality of equipment holding units 21, an equipment conveyance unit 22, object detection units 23, and a vial detection unit 24. In addition, as indicated by reference numeral 1001 in FIG. 6, the vial shelf 20 includes a first reading unit 25 and a roller driving unit 26. Although the equipment holding unit 21 is also attached to an attachment portion 221 of the equipment conveyance unit 22 illustrated in FIG. 5, illustration thereof is omitted.

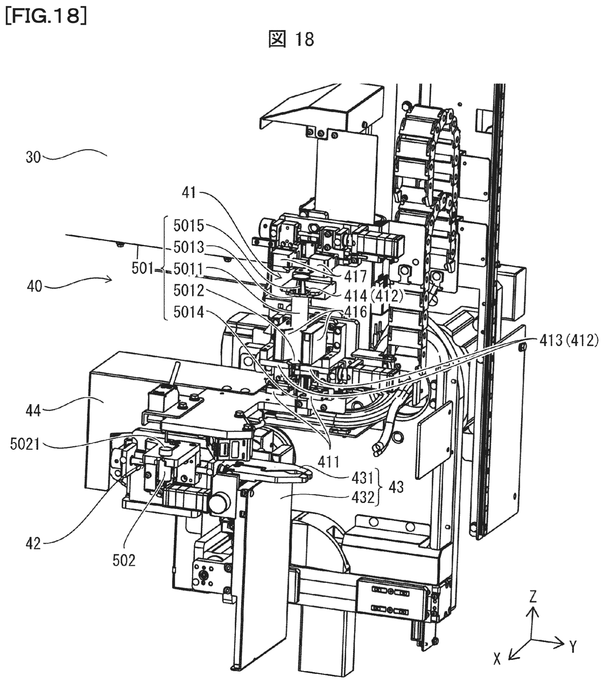

Prior to the drug-mixing processing, the vial shelf 20 is filled with the vials 502 by the user. The vial 502 is provided with a stopper (rubber stopper) 5021 (see FIG. 18) that closes an opening and a lid that covers the stopper 5021. The vial 502 is supplied to the vial shelf 20 with the lid removed. In addition, similarly to the syringe shelf 10, a plurality of holes 172 are formed in a plate-like member 171 that defines each vial shelf 20.

The equipment holding units 21 are members capable of holding the vial 502. In the at least one embodiment, one vial 502 is held in one equipment holding unit 21, but a plurality of vials 502 may be held in one equipment holding unit 21.

In the at least one embodiment, as illustrated in FIG. 6, the equipment holding unit 21 includes free rollers 211, equipment clamping units 212, a driving roller 213, and a first magnet gear 214.

The equipment clamping unit 212 clamps the vial 502. In the at least one embodiment, the equipment clamping units 212 clamp the neck portion of the vial 502. A pair of equipment clamping units 212 are biased in a direction toward each other so as to be able to clamp the vial 502. At the tip of the equipment clamping unit 212, the free roller 211 having a rotation axis extending in the up-down direction and rotatable on the XY plane is provided. As indicated by reference symbol 1002 in FIG. 6, the free rollers 211 support the neck portion of the vial 502 at three points together with the driving roller 213 provided on the side on which the equipment clamping unit 212 is pivotally supported and at a position facing the central part of the free roller 211.

The first magnet gear 214 is provided above the drive roller 213. The drive roller 213 and the first magnet gear 214 are connected to a common rotation axis extending in the up-down direction, and are rotatable on the XY plane. The drive roller 213 also rotates when the first magnet gear 214 rotates in conjunction with the rotation of a second magnet gear 261 included in the roller driving unit 26.

The equipment conveyance unit 22 is a member that conveys at least one of the plurality of equipment holding units 21 to a work area Ar2. The equipment conveyance unit 22 has the same function and structure as in the equipment conveyance unit 12. The conveyance of the equipment holding unit 21 is controlled based on the detection results obtained from the object detection units 23 and the vial detection unit 24, instead of the detection results obtained from the object detection units 13 and the syringe detection unit 14, similarly to the conveyance of the equipment holding unit 11. Further, the conveyance of the equipment holding unit 21 may be controlled based on a conveyance instruction from a user, similarly to the conveyance of the equipment holding unit 11. The work area Ar2 has the same function as in the work area Ar1, except that the object to be attached to the equipment holding unit 21 or the object to be removed from the equipment holding unit 21 is the vial 502.

Each of the object detection units 23 is a member that detects an object inserted into the work area Ar2. In the at least one embodiment, the object detection units 23 detect a user's hand or arm in a state of being inserted into the work area Ar2. The vial detection unit 24 detects the vial 502 held by the equipment holding unit 21 located in the work area Ar2.

The first reading unit 25 is a member that reads type information indicating the type of drug housed in the vial 502, which is shown on a surface of the vial 502, in a part of a conveyance path on which the equipment conveyance unit 22 conveys the plurality of equipment holding units 21. The first reading unit 25 reads the type information (for example, a barcode) at a take-out position PO11 at which the first conveyance unit 110 takes out the vial 502.

The roller driving unit 26 is a motor that rotates the second magnet gear 261 attached to the roller driving unit 26 in order to rotate the driving roller 213 of the equipment holding unit 21. When the roller driving unit 26 rotates the second magnet gear 261, the driving roller 213 rotates via the first magnet gear 214. As a result, the vial 502 in abutment against the driving roller 213 rotates. Accordingly, the first reading unit 25 can read the type information regardless of the position on the surface of the vial 502 at which the type information is provided.

### [Specific Configuration of Needle Removal Unit]

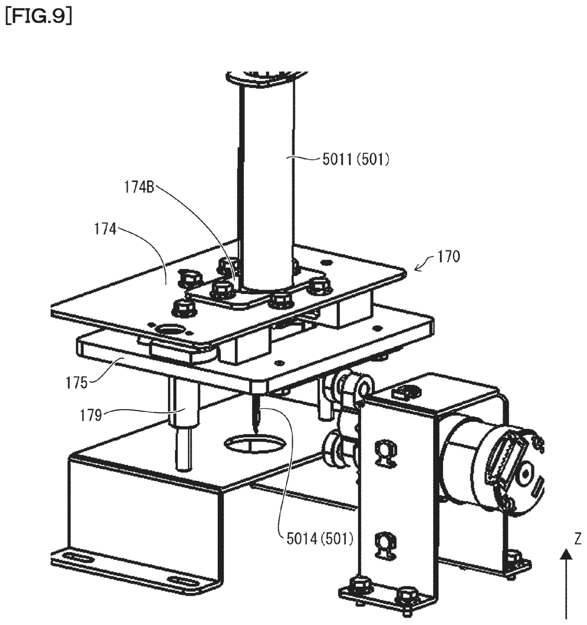

FIG. 9 is a perspective view for illustrating an example of an external appearance of the needle removal unit 170. FIG. 10 is a sectional view for illustrating an example of a cross-section of the needle removal unit 170 parallel to the insertion direction of the needle 5014 attached to the syringe 501. FIG. 10 is also a sectional view for illustrating an example of a cross-section that passes through an insertion port 174A formed in a first member 174 and is perpendicular to a rotation axis 176A. Reference numeral 1031 in FIG. 10 indicates a view for illustrating a state given before the needle removal unit 170 removes the needle 5014 from the syringe 501. Reference numeral 1032 in FIG. 10 indicates a view for illustrating a state given when the needle 5014 is removed from the syringe 501 by the needle removal unit 170.

As illustrated in FIG. 9 and FIG. 10, the needle removal unit 170 includes the first member 174, a second member 175, rotating bodies 176, a first detection unit 177, a second detection unit 178, and a driving mechanism 179.

The first member 174 is a member including: the insertion port 174A into which the insertion hub 5019 and the needle 5014 attached to the syringe 501 are allowed to be inserted; and a receiving part 174B that receives the barrel 5011. In the at least one embodiment, after the drug-mixing operation by the drug-mixing unit 40 is completed, the first conveyance unit 110 removes the syringe 501 from the drug-mixing unit 40. The first conveyance unit 110 conveys the syringe 501 removed from the drug-mixing unit 40, to the needle removal unit 170, and inserts the insertion hub 5019 and the needle 5014 of the syringe 501 into the insertion port 174A. As illustrated in FIG. 10, a part of the syringe 501 (specifically, the barrel tip 5017) is inserted into the insertion port 174A.

At the time when the needle 5014 of the syringe 501 is inserted into the insertion port 174A (when the needle 5014 is removed from the syringe 501), the needle cap 5016 has not been attached to the needle 5014. The needle cap 5016 attached to the needle 5014 of the syringe 501 is removed from the syringe 501 by the cap removal unit 180 before the removal of the needle 5014. The cap removal unit 180 removes the needle cap 5016 from the syringe 501, for example, before the drug-mixing operation (before puncturing the infusion container 503 or the vial 502 with the needle 5014).

The shape of the receiving part 174B is a concave shape that substantially matches the shape of the bottom part 5018 (see FIG. 7), at which the barrel tip 5017 is provided, of the barrel 5011. Accordingly, under a state in which the insertion hub 5019 and the needle 5014 of the syringe 501 are inserted into the insertion port 174A, the bottom part 5018 of the barrel 5011 can be stably brought into abutment against the receiving part 174B. Thus, the posture of the syringe 501 having the insertion hub 5019 and the needle 5014 inserted into the insertion port 174A can be stabilized. The shape of the receiving part 174B is a mortar shape. The insertion port 174A is provided at the bottom of the receiving part 174B, and may have any shape that allows insertion of the insertion hub 5019.

Further, when a claw part 176B of the rotating body 176 applies a force to the flange 5020 of the insertion hub 5019 in the insertion direction of the needle 5014 attached to the syringe 501, the receiving part 174B holds the barrel 5011 so that the barrel 5011 does not move in the insertion direction.

The second member 175 is a member that faces a surface of the first member 174 opposite to a surface into which the needle 5014 attached to the syringe 501 is to be inserted, and moves so as to approach the first member 174 or moves so as to be separated from the first member 174. In the at least one embodiment, the second member 175 moves in the up-down direction. The first conveyance unit 110 inserts the insertion hub 5019 and the needle 5014 of the syringe 501 into the insertion port 174A under a state in which the second member 175 is located at a position farthest from the first member 174.

The rotating body 176 includes: the rotation axis 176A extending in a direction perpendicular to the insertion direction of the needle 5014 attached to the syringe 501; and the claw part 176B capable of abutting against the flange 5020 of the insertion hub 5019 inserted from the insertion port 174A. The rotating body 176 rotates about the rotation axis 176A to bring the claw part 176B into abutment against the flange 5020, and to apply a force to the flange 5020 in the insertion direction of the needle 5014. Accordingly, the needle removal unit 170 removes the insertion hub 5019 and the needle 5014 from the syringe 501. As described above, the needle removal unit 170 can autonomously remove the needle 5014 inserted into the insertion port 174A, from the syringe 501.

Further, the rotating body 176 includes an insertion part 176C inserted into an elongated hole 175A formed in the upper part of the second member 175. The elongated hole 175A is a hole portion extending in a direction perpendicular to the insertion direction of the needle 5014 and the extending direction of the rotation axis 176A. The insertion part 176C is fixed to the rotating body 176. The rotating body 176 rotates about the rotation axis 176A as the second member 175 moves in the insertion direction of the needle 5014 or in the direction opposite thereto. The insertion part 176C is slidable in the elongated hole 175A in the extending direction of the elongated hole 175A. As the insertion part 176C slides in the elongated hole 175A, the rotating body 176 can rotate about the rotation axis 176A.

As indicated by reference numeral 1031 in FIG. 10, consideration is made on a case in which, under a state in which the insertion hub 5019 and the needle 5014 are inserted into the insertion port 174A, the second member 175 moves in the direction opposite to the insertion direction of the needle 5014 (moves so as to approach the first member 174). The second member 175 abuts against a part of the rotating body 176, and rotates the rotating body 176 about the rotation axis 176A so that the claw part 176B moves in the insertion direction of the needle 5014 (direction toward the second member 175). The second member 175 may be in abutment against a part of the rotating body 176 even at the position farthest from the first member 174.

As indicated by reference numeral 1032 in FIG. 10, the claw part 176B abuts against the flange 5020 of the insertion hub 5019 by moving in the insertion direction of the needle 5014. Then, as the rotating body 176 continues to rotate so that the claw part 176B moves in the insertion direction of the needle 5014, the claw part 176B in abutment against the flange 5020 continues to push the flange 5020 toward the second member 175 side. With this pushing by the claw part 176B, the insertion hub 5019 having the needle 5014 attached thereto can be removed from the syringe 501.

The position of the rotating body 176 given under a state in which the second member 175 is present at the position farthest from the first member 174 is defined so that the insertion hub 5019 and the needle 5014 are not brought into contact with the claw part 176B when the insertion hub 5019 and the needle 5014 are inserted into the insertion port 174A. Further, the position of the rotating body 176 is defined so as to allow the claw part 176B to abut against the flange 5020 such that the insertion hub 5019 inserted from the insertion port 174A can be removed from the barrel tip 5017 when the rotating body 176 rotates.

The first detection unit 177 is a detection unit that detects the barrel 5011 of the syringe 501 when the insertion hub 5019 and the needle 5014 are inserted into the insertion port 174A. The first detection unit 177 may be, for example, an optical sensor including a light receiving unit that receives light reflected by the barrel 5011. The optical sensor may include a light emitting unit that emits light. Further, the first detection unit 177 may be an image pickup unit (camera) that captures an image of the barrel 5011, instead of the optical sensor. Other detection units may also be achieved by an optical sensor that receives light reflected by an object, or an image pickup unit that captures an image of the object.

The second detection unit 178 is a detection unit that detects the needle 5014 inserted from the insertion port 174A. When the second detection unit 178 detects the needle 5014, the needle removal control unit 154 moves the second member 175 in the direction opposite to the insertion direction of the needle 5014, and rotates the rotating body 176 about the rotation axis 176A. The second detection unit 178 may function as a detection unit that detects the insertion hub 5019 inserted from the insertion port 174A.

In the at least one embodiment, when the first detection unit 177 detects the barrel 5011, and the second detection unit 178 detects the needle 5014 or the insertion hub 5019, the needle removal control unit 154 moves the second member 175 in the direction opposite to the insertion direction of the needle 5014. After that, when the second detection unit 178 no longer detects the needle 5014 or the insertion hub 5019, the needle removal control unit 154 moves the second member 175 in the insertion direction of the needle 5014, and moves the second member 175 to its original initial position. Further, the first conveyance control unit 143 controls the first conveyance unit 110 to remove the syringe 501 (the barrel 5011 and the plunger 5015) from which the insertion hub 5019 and the needle 5014 have been removed, from the needle removal unit 170.

The drug-mixing apparatus 1 is only required to include at least the second detection unit 178. In this case, the control unit 140 may execute the above-mentioned processing based on whether the needle 5014 or the insertion hub 5019 is detected by the second detection unit 178.

The driving mechanism 179 is a mechanism that is connected to the second member 175 and moves the second member 175 with respect to the first member 174 along the insertion direction of the needle 5014 or in the direction opposite thereto under the control of the needle removal control unit 154. When the second detection unit 178 detects the needle 5014 or the insertion hub 5019, the needle removal control unit 154 controls the driving mechanism 179 to move the second member 175 in the direction opposite to the insertion direction of the needle 5014. Further, when the second detection unit 178 no longer detects the needle 5014 or the insertion hub 5019, the needle removal control unit 154 controls the driving mechanism 179 to move the second member 175 in the insertion direction of the needle 5014.

The driving mechanism 179 may be a mechanism that is connected to the first member 174 instead of the second member 175, and moves the first member 174 with respect to the second member 175 along the insertion direction of the needle 5014 or in the direction opposite thereto. In this case, as the rotating body 176 rotates about the rotation axis 176A along with the movement of the first member 174, the claw part 176B abuts against the flange 5020 to apply a force to the flange 5020 in the insertion direction of the needle 5014.

As described above, the drug-mixing apparatus 1 includes: the first member 174 that holds the syringe 501 having the needle 5014 attached thereto; and the second detection unit 178 that detects the needle 5014 of the syringe 501 held by the first member 174. The drug-mixing apparatus 1 further includes a removal unit that removes the needle 5014 from the syringe 501 when the second detection unit 178 detects the needle 5014. The removal unit may include, for example, the first member 174 or the second member 175 that can move relatively, the rotating bodies 176, and the driving mechanism 179.

### <Positional Relationship between Needle Removal Unit and Waste Box>

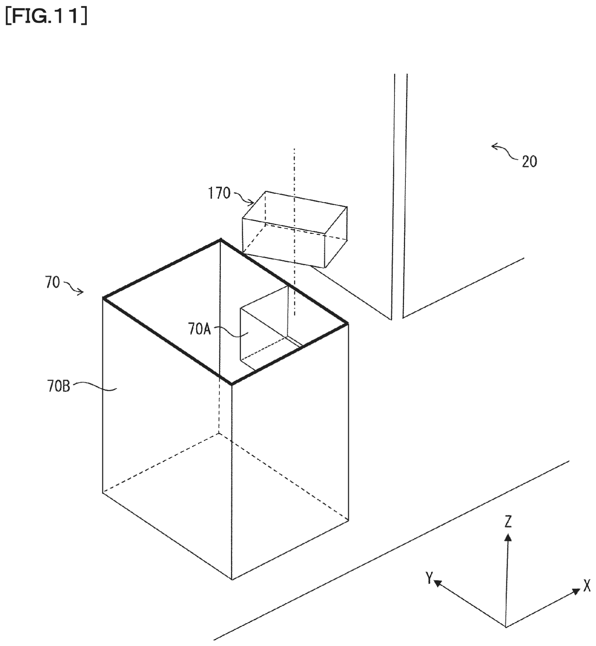

FIG. 11 is a schematic perspective view for illustrating an example of the positional relationship between the needle removal unit 170 and the waste box 70. As illustrated in FIG. 11, in the at least one embodiment, the drug-mixing apparatus 1 includes, as the waste box 70, a first housing unit 70A that houses the needle 5014, and a second housing unit 70B that houses the syringe 501 having no needle 5014 attached thereto. The waste box 70 is provided on the front side inside the drug-mixing apparatus 1 in consideration of ease of takeout. In the at least one embodiment, the first housing unit 70A is provided on the front side inside the second housing unit 70B.

In the at least one embodiment, the insertion hub 5019 and the needle 5014 removed by the needle removal unit 170 drop due to own weights of the insertion hub 5019 and the needle 5014. Accordingly, the needle removal unit 170 is provided above the first housing unit 70A. Thus, the needle 5014 having the insertion hub 5019 attached thereto, which has been removed from the syringe 501 by the needle removal unit 170, is discarded into the first housing unit 70A.

In the at least one embodiment, after the insertion hub 5019 and the needle 5014 are removed from the syringe 501 by the needle removal unit 170, the first conveyance unit 110 conveys the syringe 501 having no insertion hub 5019 and no needle 5014 attached thereto (the barrel 5011 and the plunger 5015) to the second housing unit 70B. Accordingly, the syringe 501 from which the needle 5014 having the insertion hub 5019 attached thereto has been removed by the needle removal unit 170 is discarded into the second housing unit 70B.

As described above, the drug-mixing apparatus 1 can separately discard the needle 5014 having the insertion hub 5019 attached thereto, and the syringe 501 having no insertion hub 5019 and no needle 5014 attached thereto.

Further, the volume of the syringe 501 is larger than the volume of the needle 5014. Accordingly, in the case of discarding the syringe 501 having the needle 5014 attached thereto into a predetermined space, the number of the syringes 501 is smaller than the number of the needles 5014 in the case of discarding the needle 5014 into the predetermined space. Further, in the case of discarding the syringe 501 having the needle 5014 attached thereto, the disposal cost is equivalent to that in the case of discarding the needle 5014. Accordingly, in the case of discarding the syringe 501 into the predetermined space, although the number of syringes 501 having the needles 5014 attached thereto is smaller than the number of needles 5014 in the case of discarding the needle 5014, the disposal cost is equivalent to that in the case of discarding the needle 5014.

The disposal cost of the needle 5014 is higher than the disposal cost of the members of the syringe 501 other than the needle 5014. Accordingly, in the case of discarding the syringe 501 having the needle 5014 attached thereto into a predetermined space, the disposal cost is higher than in the case of discarding the syringe 501 having no needle 5014 attached thereto.

In the at least one embodiment, the needle removal unit 170 removes the needle 5014 from the syringe 501, and thus the syringe 501 having no needle 5014 attached thereto can be discarded. Accordingly, the disposal cost can be reduced as compared to the case of discarding the syringe 501 having the needle 5014 attached thereto.

In the at least one embodiment, the needle removal unit 170 is provided above the first housing unit 70A, but the needle removal unit 170 is not always required to be provided above the first housing unit 70A. In this case, for example, the first conveyance unit 110 may hold the insertion hub 5019 and the needle 5014 removed by the needle removal unit 170, and may convey and discard the insertion hub 5019 and the needle 5014 to the first housing unit 70A. Further, in this case, the syringe 501 from which the insertion hub 5019 and the needle 5014 have been removed may be dropped into the second housing unit 70B by the own weight of the syringe 501. In this case, the needle removal unit 170 may be provided above the second housing unit 70B.

Further, the needle removal unit 170 may be any mechanism that can remove the needle 5014 from the syringe 501. For example, the needle removal unit 170 may have a mechanism that pulls out only the needle 5014 from the syringe 501. In this case, only the needle 5014 is discarded into the first housing unit 70A, and the syringe 501 having no needle 5014 attached thereto (the barrel 5011, the plunger 5015, and the insertion hub 5019) is discarded into the second housing unit 70B. When the needle removal unit 170 has a mechanism that pulls out only the needle 5014 from the syringe 501, the shape of the insertion port 174A may be any shape that allows insertion of the needle 5014.

The needle cap 5016 removed by the cap removal unit 180 is discarded into the second housing unit 70B. When the drug-mixing apparatus 1 includes a third housing unit different from the first housing unit 70A and the second housing unit 70B, the needle cap 5016 removed by the cap removal unit 180 may be discarded into the third housing unit. As described above, the needle 5014 and the needle cap 5016 are discarded into separate spaces, and thus the needle 5014 and the needle cap 5016 can be separately collected. Further, the vial 502 used in the drug-mixing operation may be discarded into the second housing unit 70B.

### <Operation Example of Drug-mixing Apparatus>

### (Operation Example in Case of Drug-mixing Apparatus Including Needle Removal Unit)

When the drug-mixing apparatus 1 includes the needle removal unit 170, the drug-mixing apparatus 1 operates, for example, as follows.

The first conveyance unit 110 takes out the syringe 501 from the syringe shelf 10, and then conveys the syringe 501 to the cap removal unit 180. The cap removal unit 180 removes the needle cap 5016 from the syringe 501. The first conveyance unit 110 conveys the syringe 501 from which the needle cap 5016 has been removed, to the drug-mixing unit 40. Further, the first conveyance unit 110 takes out the needle cap 5016 removed by the cap removal unit 180, from the cap removal unit 180, and conveys and discards the needle cap 5016 to the second housing unit 70B.

After the drug-mixing operation by the drug-mixing unit 40, the first conveyance unit 110 conveys the syringe 501 used in the drug-mixing operation to the needle removal unit 170. The needle removal unit 170 removes the needle 5014 from the syringe 501 used in the drug-mixing operation. The first conveyance unit 110 does not hold the syringe 501 at the time when the needle removal unit 170 removes the needle 5014. The first conveyance unit 110 places the barrel 5011 on the receiving part 174B of the first member 174, and then releases the holding of the syringe 501. In the at least one embodiment, the first member 174 on which the barrel 5011 is placed does not move, and hence, the first conveyance unit 110 is not required to hold the syringe 501 at the time when the needle removal unit 170 removes the needle 5014. After that, as described above, the needle 5014 removed from the syringe 501 is discarded into the first housing unit 70A, and the syringe 501 from which the needle 5014 has been removed is discarded into the second housing unit 70B.

### (Operation Example in Case of Drug-mixing Apparatus Including No Needle Removal Unit)

In the at least one embodiment, unless otherwise specified, description is given of a case in which the drug-mixing apparatus 1 includes the needle removal unit 170, but the drug-mixing apparatus 1 is not always required to include the needle removal unit 170. In this case, the waste box 70 is not required to include the first housing unit 70A. When the drug-mixing apparatus 1 does not include the needle removal unit 170, the drug-mixing apparatus 1 operates, for example, as follows.

The first conveyance unit 110 takes out the syringe 501 from the syringe shelf 10, and then conveys the syringe 501 to the cap removal unit 180. The cap removal unit 180 removes the needle cap 5016 from the syringe 501. The first conveyance unit 110 conveys the syringe 501 from which the needle cap 5016 has been removed, to the drug-mixing unit 40. The cap removal unit 180 holds the needle cap 5016 until the drug-mixing operation using the syringe 501 is completed.

After the completion of the drug-mixing operation, the first conveyance unit 110 conveys the syringe 501 used in the drug-mixing operation, to the cap removal unit 180. The cap removal unit 180 attaches the needle cap 5016 held by the cap removal unit 180, to the needle 5014 of this syringe 501. That is, the cap removal unit 180 functions as a cap attaching/detaching unit that attaches and detaches the needle cap 5016 to and from the needle 5014 of the syringe 501. After that, the first conveyance unit 110 conveys the syringe 501 having the needle cap 5016 attached thereto, to the waste box 70.

### (Brief Summary)

When the drug-mixing apparatus 1 includes the needle removal unit 170, the drug-mixing apparatus 1 is not required to perform an operation of attaching the needle cap 5016 to the needle 5014 of the syringe 501 used in the drug-mixing operation, before discarding the syringe 501 used in the drug-mixing operation. Accordingly, the drug-mixing apparatus 1 can shorten the time required for the drug-mixing operation by an amount corresponding to the time taken for performing the above-mentioned operation.

Further, when the needle 5014 used in the drug-mixing operation is bent, the cap removal unit 180 may not be able to perform the operation of attaching the needle cap 5016 to the needle 5014. When the drug-mixing apparatus 1 includes the needle removal unit 170, it is not required to perform the above-mentioned operation. Accordingly, a situation in which such an error occurs can be avoided.

Further, when the drug-mixing apparatus 1 includes the needle removal unit 170, the cap removal unit 180 is not required to include a mechanism for attaching the needle cap 5016 to the needle 5014 of the syringe 501 used in the drug-mixing operation.

However, even when the needle removal unit 170 is provided, the cap removal unit 180 may include a mechanism for attaching the needle cap 5016 to the needle 5014 of the syringe 501 used in the drug-mixing operation. In this case, the cap removal unit 180 holds the needle cap 5016 removed before the drug-mixing operation, and attaches the needle cap 5016 to the needle 5014 after the drug-mixing operation. After that, the needle removal unit 170 removes the needle 5014 together with the needle cap 5016 from the syringe 501. Accordingly, the barrel 5011 and the plunger 5015, and the needle 5014 having the needle cap 5016 attached thereto can be separately discarded.

Even under a state in which the needle cap 5016 is attached to the needle 5014, the flange 5020 is not covered with the needle cap 5016. Accordingly, the needle removal unit 170 can remove the needle 5014 together with the needle cap 5016 from the syringe 501, by bringing the claw part 176B into abutment against the flange 5020 to apply a force in the insertion direction of the needle 5014.

### <Modification Example of Needle Removal Unit>

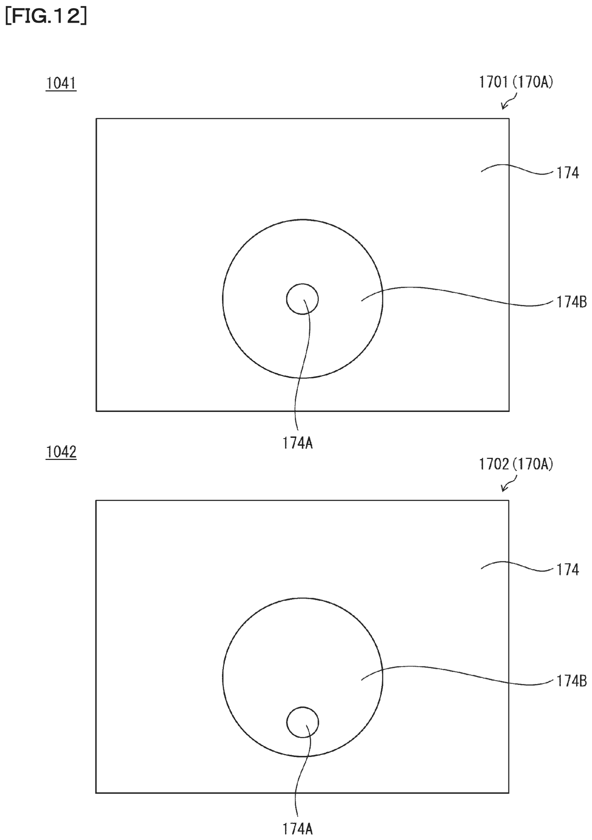

FIG. 12 is a view for illustrating a needle removal unit 170A that is a modification example of the needle removal unit 170. Reference numeral 1041 in FIG. 12 indicates a plan view for schematically illustrating an example of a first needle removal unit 1701 (first needle removal part). Reference numeral 1042 in FIG. 12 indicates a plan view for schematically illustrating an example of a second needle removal unit 1702 (second needle removal part).

The first needle removal unit 1701 and the second needle removal unit 1702 have the same function as the needle removal unit 170. The shape of the first needle removal unit 1701 is the same as that of the needle removal unit 170. Meanwhile, the shape of the second needle removal unit 1702 is the same as that of the needle removal unit 170, except that the shape of the receiving part 174B (arrangement position of the insertion port 174A) provided in the first member 174 is different. Accordingly, FIG. 12 shows only plan views of the first needle removal unit 1701 and the second needle removal unit 1702 (only plan views of the first members 174).

When the drug-mixing apparatus 1 executes a drug-mixing operation using the centric-tip syringe 501A and the eccentric-tip syringe 501B, the needle removal unit 170A may include the first needle removal unit 1701 and the second needle removal unit 1702. The first needle removal unit 1701 is a member that removes the needle 5014 attached to the centric-tip syringe 501A, from the centric-tip syringe 501A. The second needle removal unit 1702 is a member that removes the needle 5014 attached to the eccentric-tip syringe 501B, from the eccentric-tip syringe 501B.

In the first needle removal unit 1701, the shape of the receiving part 174B is a mortar shape. Then, as indicated by reference numeral 1041 in FIG. 12, when the receiving part 174B is viewed in a plan view, the insertion port 174A is formed at the center of the receiving part 174B.

That is, the shape of the receiving part 174B and the position of the insertion port 174A correspond to a concave shape that substantially matches the shape of the bottom part 5018 (end portion at which the barrel tip 5017 is provided) of the barrel 5011 included in the centric-tip syringe 501A. Accordingly, under a state in which the insertion hub 5019 and the needle 5014 of the centric-tip syringe 501A are inserted into the insertion port 174A, the bottom part 5018 of the barrel 5011 of the centric-tip syringe 501A can be stably brought into abutment against the receiving part 174B.

Also in the second needle removal unit 1702, the shape of the receiving part 174B is a mortar shape. However, as indicated by reference numeral 1042 in FIG. 12, when the receiving part 174B is viewed in a plan view, the insertion port 174A is formed at a position deviated from the center of the receiving part 174B.

That is, the shape of the receiving part 174B and the position of the insertion port 174A correspond to a concave shape that substantially matches the shape of the bottom part 5018 (end portion at which the barrel tip 5017 is provided) of the barrel 5011 included in the eccentric-tip syringe 501B. Accordingly, under a state in which the insertion hub 5019 and the needle 5014 of the eccentric-tip syringe 501B are inserted into the insertion port 174A, the bottom part 5018 of the barrel 5011 of the eccentric-tip syringe 501B can be stably brought into abutment against the receiving part 174B.

Accordingly, even when the drug-mixing apparatus 1 uses the centric-tip syringe 501A and the eccentric-tip syringe 501B for the drug-mixing operation, the postures of the centric-tip syringe 501A and the eccentric-tip syringe 501B, in each of which the insertion hub 5019 and the needle 5014 have been inserted into the insertion port 174A, can be stabilized. Accordingly, the needle removal unit 170A can remove the needle 5014 from each of the centric-tip syringe 501A and the eccentric-tip syringe 501B.

In this modification example, as illustrated in FIG. 4, the control unit 140 may include the determination unit 151. The determination unit 151 determines whether the syringe 501 used for the drug-mixing operation is a centric-tip syringe 501A or an eccentric-tip syringe 501B. Based on the determination result obtained from the determination unit 151, the first conveyance control unit 143 controls the first conveyance unit 110 to convey the centric-tip syringe 501A used in the drug-mixing operation to the first needle removal unit 1701, and convey the eccentric-tip syringe 501B used in the drug-mixing operation to the second needle removal unit 1702.

### <Determination of Type of Syringe>

The determination unit 151 may determine the type of the syringe 501 held by the equipment holding unit 11, based on the detection result obtained from the needle detection unit 15, the barrel tip detection unit 18, or the barrel detection unit 17, which is provided in the syringe shelf 10.

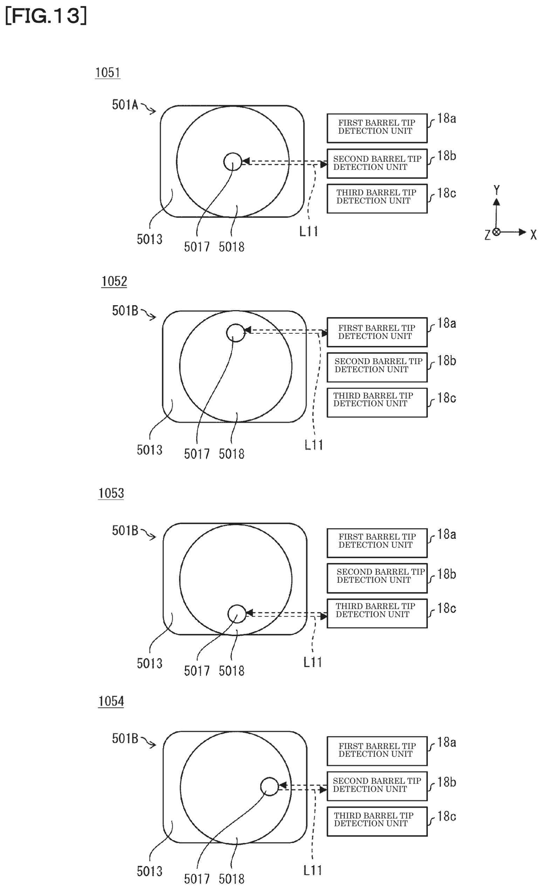

FIG. 13 is a schematic view for illustrating an example of the positional relationship between the barrel tip 5017 of the syringe 501 held by the equipment holding unit 11 and the barrel tip detection unit 18. FIG. 13 is a schematic view of the syringe 501 held by the equipment holding unit 11 in the work area Ar1, as viewed from below.

As illustrated in FIG. 13, the barrel tip detection unit 18 may include a first barrel tip detection unit 18a, a second barrel tip detection unit 18b, and a third barrel tip detection unit 18c. In FIG. 13, the first barrel tip detection unit 18a side is the deep direction (+Y-axis direction), and the syringe side door 16 is provided on the third barrel tip detection unit 18c side.

As indicated by reference numeral 1051 in FIG. 13, when the centric-tip syringe 501A is held by the equipment holding unit 11, the second barrel tip detection unit 18b detects light L11 reflected by the barrel tip 5017 of the centric-tip syringe 501A. As indicated by reference numeral 1052 and reference numeral 1053 in FIG. 13, when the eccentric-tip syringe 501B is held by the equipment holding unit 11, the first barrel tip detection unit 18a and the third barrel tip detection unit 18c detect light L11 reflected by the barrel tip 5017 of the eccentric-tip syringe 501B. The second barrel tip detection unit 18b detects the light L11 reflected by the barrel tip 5017 of the eccentric-tip syringe 501B, when the eccentric-tip syringe 501B is held by the equipment holding unit 11 under a state of being rotated by 90 degrees about its axis from each of the states indicated by reference numeral 1052 and reference numeral 1053 in FIG. 13.

Reference numeral 1054 in FIG. 13 indicates an example of the above-mentioned positional relationship given when the eccentric-tip syringe 501B is held by the equipment holding unit 11 under a state of being rotated by 90 degrees about its axis from each of the states indicated by reference numeral 1052 and reference numeral 1053 in FIG. 13. Reference numeral 1054 in FIG. 13 indicates a case in which, when the eccentric-tip syringe 501B is viewed from below, the barrel tip 5017 is located on the second barrel tip detection unit 18b side from the center of the bottom part 5018 of the barrel 5011. There is also a case in which, under a state in which the eccentric-tip syringe 501B has been rotated by 90 degrees about its axis from each of the states indicated by reference numeral 1052 and reference numeral 1053 in FIG. 13, when the eccentric-tip syringe 501B is viewed from below, the barrel tip 5017 is located at a position distant from the second barrel tip detection unit 18b across the center of the bottom part 5018.

Accordingly, when the first barrel tip detection unit 18a or the third barrel tip detection unit 18c detects the light L11, the determination unit 151 determines that the syringe 501 held by the equipment holding unit 11 is an eccentric-tip syringe 501B.

Here, a first time, which is taken from when the light emitted by the second barrel tip detection unit 18b is reflected by the centric-tip syringe 501A until when the light returns to the second barrel tip detection unit 18b, and a second time, which is taken from when the light is reflected by the eccentric-tip syringe 501B until when the light returns to the second barrel tip detection unit 18b, are different from each other.

Consideration is made on a case in which the eccentric-tip syringe 501B is held by the equipment holding unit 11 under the state indicated by reference numeral 1054 in FIG. 13. In this case, the distance between the barrel tip 5017 of the eccentric-tip syringe 501B and the second barrel tip detection unit 18b is shorter than the distance between the barrel tip 5017 of the centric-tip syringe 501A in the case of being held by the equipment holding unit 11 and the second barrel tip detection unit 18b. Accordingly, when the eccentric-tip syringe 501B is held by the equipment holding unit 11 under the state indicated by reference numeral 1054 in FIG. 13, the second time is shorter than the first time.

Meanwhile, consideration is made on a case in which the eccentric-tip syringe 501B is held by the equipment holding unit 11 under a state in which, when the eccentric-tip syringe 501B is viewed from below, the barrel tip 5017 is located at a position distant from the second barrel tip detection unit 18b across the center of the bottom part 5018. In this case, the distance between the barrel tip 5017 of the eccentric-tip syringe 501B and the second barrel tip detection unit 18b is longer than the distance between the barrel tip 5017 of the centric-tip syringe 501A in the case of being held by the equipment holding unit 11 and the second barrel tip detection unit 18b. Thus, when the eccentric-tip syringe 501B is held by the equipment holding unit 11 under a state in which the barrel tip 5017 is located at a position distant from the second barrel tip detection unit 18b across the center of the bottom part 5018, the second time is longer than the first time.

Accordingly, when the second barrel tip detection unit 18b detects the light L11, the determination unit 151 can determine whether the syringe 501 held by the equipment holding unit 11 is a centric-tip syringe 501A or an eccentric-tip syringe 501B, based on the time taken from when the emitted light is reflected by the syringe 501 until when the light returns to the second barrel tip detection unit 18b.

Specifically, the control unit 140 measures the time taken from when the light emitted by the second barrel tip detection unit 18b is reflected by the syringe 501 until the light returns to the second barrel tip detection unit 18b. When the second barrel tip detection unit 18b has detected the light L11, the determination unit 151 determines whether the syringe 501 held by the equipment holding unit 11 is a centric-tip syringe 501A or an eccentric-tip syringe 501B by determining whether the measured time falls between a first threshold value T11 and a second threshold value T12.

Specifically, when the determination unit 151 determines that the measured time falls between the first threshold value T11 and the second threshold value T12 (when T11≤measured time≤T12 is satisfied), the determination unit 151 determines that the syringe 501 held by the equipment holding unit 11 is a centric-tip syringe 501A. Meanwhile, when the determination unit 151 determines that the measured time is less than the first threshold value T11 or more than the second threshold value T12 (when measured time<T11 or T12<measured time is satisfied), the determination unit 151 determines that the syringe 501 held by the equipment holding unit 11 is an eccentric-tip syringe 501B.

Information indicating the first threshold value T11 and the second threshold value T12 is stored in the storage unit 200. The first threshold value T11 and the second threshold value T12 are only required to be determined, for example, through experiments, so that, when the second barrel tip detection unit 18b has detected the light L11, it can be determined whether the syringe 501 held by the equipment holding unit 11 is a centric-tip syringe 501A or an eccentric-tip syringe 501B.

Further, three needle detection units 15 may be provided as in the barrel tip detection unit 18. In this case, the determination unit 151 may determine whether the syringe 501 held by the equipment holding unit 11 is a centric-tip syringe 501A or an eccentric-tip syringe 501B, based on the detection results obtained from the three needle detection units 15.

Further, for example, information indicating the speed of the equipment conveyance unit 12 in front of the barrel detection unit 17, and information indicating the thicknesses of various types of barrels 5011 that can be used in drug-mixing are stored in the storage unit 200. The control unit 140 calculates the thickness of the barrel 5011 that has passed in front of the barrel detection unit 17, based on the detection time of the barrel 5011 detected by the barrel detection unit 17 and the speed of the equipment conveyance unit 12. Accordingly, the control unit 140 can identify the thickness of the barrel 5011 that has passed in front of the barrel detection unit 17 (the type of the syringe 501 held by the equipment holding unit 11).

As described above, the thicknesses of the centric-tip syringe 501A and the eccentric-tip syringe 501B to be used in the drug-mixing apparatus 1 may be different from each other. In this case, the determination unit 151 can determine whether the syringe 501 held by the equipment holding unit 11 is a centric-tip syringe 501A or an eccentric-tip syringe 501B, based on the thickness of the barrel 5011 that has passed in front of the barrel detection unit 17.

Further, for example, the drug-mixing apparatus 1 may include a camera that captures an image of the syringe 501 held by the equipment holding unit 11. In this case, for example, an image of the centric-tip syringe 501A and an image of the eccentric-tip syringe 501B are stored in the storage unit 200. The determination unit 151 may determine whether the syringe 501 held by the equipment holding unit 11 is a centric-tip syringe 501A or an eccentric-tip syringe 501B, based on the image captured by the camera and the images stored in the storage unit 200.

In the at least one embodiment, the determination unit 151 determines whether the syringe 501 held by the equipment holding unit 11 is a centric-tip syringe 501A or an eccentric-tip syringe 501B. However, the configuration is not limited thereto, and the determination unit 151 may determine, for example, whether the syringe 501 held by a syringe holding unit 41 (see FIG. 18) of the drug-mixing unit 40 is a centric-tip syringe 501A or an eccentric-tip syringe 501B. In this case, for the determination by the determination unit 151, a detection unit that detects the barrel 5011, the needle 5014, or the barrel tip 5017 is provided at a position at which the detection unit can detect the barrel 5011, the needle 5014, or the barrel tip 5017 of the syringe 501 held by the drug-mixing unit 40.

### [Specific Configuration of First Conveyance Unit]

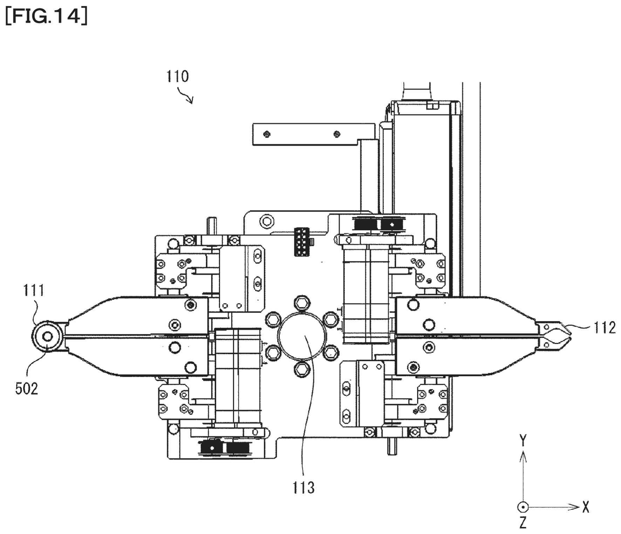

FIG. 14 is a view for illustrating an example of the configuration of the first conveyance unit 110. As illustrated in FIG. 14, it includes a first claw part 111, a second claw part 112, and a rotation shaft part 113.

The first claw part 111 and the second claw part 112 are members that clamp the upper part of the barrel 5011 of the syringe 501, or the neck portion of the vial 502, respectively. The rotation shaft part 113 is a shaft part extending in the up-down direction, and rotates the first claw part 111 and the second claw part 112 on the XY plane.

The first claw part 111 and the second claw part 112 may have any structure that can hold the syringe 501 and the vial 502, respectively. However, in the at least one embodiment, the drug-mixing unit 40 holds the lower part of the barrel 5011 of the syringe 501, and the body of the vial 502. Accordingly, in the at least one embodiment, the first conveyance unit 110 is configured to grip the upper part of the barrel 5011 of the syringe 501, or the neck portion of the vial 502. Thus, a position at which the drug-mixing unit 40 clamps the syringe 501 or the vial 502 from both sides in the radial direction, and a position at which the first conveyance unit 110 clamps the syringe 501 or the vial 502 from both sides in the radial direction can be made different.

For example, one of the first claw part 111 and the second claw part 112 is caused to function as a claw part that holds a new syringe 501 or vial 502 to be used for the next drug-mixing operation. That is, one claw part is caused to function as a claw part that is used in conveying the syringe 501 or the vial 502 from the syringe shelf 10 or the vial shelf 20 to the drug-mixing unit 40.

For example, another one of the first claw part 111 and the second claw part 112 is caused to function as a claw part that holds the syringe 501 or the vial 502 used in the drug-mixing operation. That is, another claw part is caused to function as a claw part that is used in conveying the syringe 501 or the vial 502 from the drug-mixing unit 40 to the waste box 70 or the needle removal unit 170.

Whether the first claw part 111 and the second claw part 112 are caused to function as a claw part that holds the syringe 501 or the vial 502 to be used for the next drug-mixing operation, or to function as a claw part that holds the syringe 501 or the vial 502 used in the drug-mixing operation may be set in advance.

In the at least one embodiment, the first conveyance unit 110 includes the first claw part 111 and the second claw part 112 as equipment holding units that hold the syringe 501 and the vial 502, but the first conveyance unit 110 may include three or more equipment holding units. That is, the first conveyance unit 110 may include a plurality of equipment holding units.

Further, when one of the plurality of equipment holding units is out of order, the conveyance of the syringe 501 and the vial 502 is performed using another one of the plurality of equipment holding units. For example, when the first claw part 111 is out of order, the conveyance of the syringe 501 and the vial 502 is performed using the second claw part 112, regardless of the above-mentioned setting. Thus, the overall operation associated with drug-mixing becomes slower as compared to the case in which the conveyance of the syringe 501 and the vial 502 is performed using a plurality of equipment holding units.

Accordingly, when the control unit 140 determines that any of the plurality of equipment holding units is out of order, the touch panel control unit 150 may notify the touch panel 80 that the operation is slowing down. In this case, the user can recognize that the drug-mixing apparatus 1 is operating in a slow mode. Thus, the drug-mixing apparatus 1 can prevent the user from worrying about the slow operation.

Further, for example, the first conveyance unit 110 may include a detection unit that detects the syringe 501 or the vial 502 held by each of the equipment holding units. The first conveyance control unit 143 may determine that the equipment holding unit is out of order when the detection unit does not detect the syringe 501 or the vial 502 after performing an operation of causing the equipment holding unit to hold the syringe 501 or the vial 502.

### [Specific Configuration of Second Conveyance Unit]

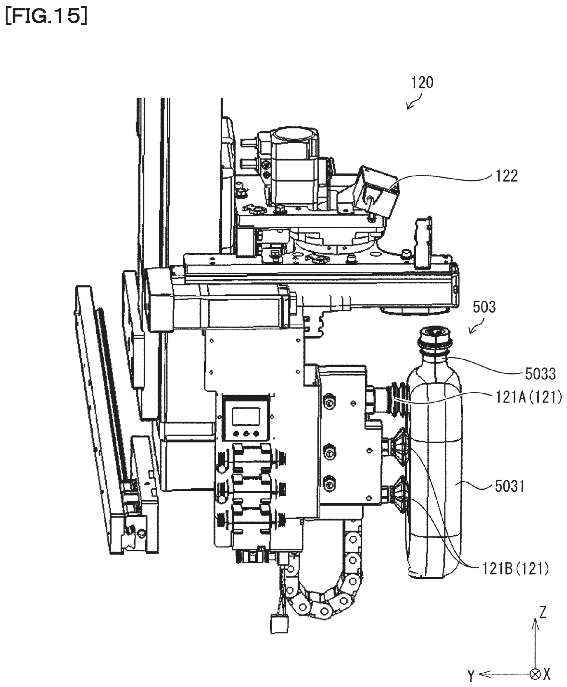

FIG. 15 is a view for illustrating an example of the configuration of the second conveyance unit 120. As illustrated in FIG. 15, the second conveyance unit 120 includes a suction unit 121 and a third reading unit 122.

The suction unit 121 is a member that suctions a body 5031 of the infusion container 503. The suction unit 121 is an example of a holding member that holds the infusion container 503, and as long as the second conveyance unit 120 can hold the infusion container 503, the holding member is not required to be the suction unit 121. The second conveyance control unit 145 controls the air pressure in the suction unit 121 to attach and detach the infusion container 503.

In the at least one embodiment, three suction units 121 are provided along the up-down direction at positions facing the infusion container 503 in the second conveyance unit 120. The three suction units 121 include a main suction unit 121A and two sub suction units 121B. The suction unit 121 may include a plurality of main suction units 121A, or may include only one sub suction unit 121B or three or more sub suction units 121B.

The main suction unit 121A is always used to suction the infusion container 503 at the time when the second conveyance unit 120 holds the infusion container 503. The main suction unit 121A is provided at a position at which the main suction unit 121A can face all of a plurality of types of infusion containers 503 having different sizes that can be housed in the drug-mixing apparatus 1. The two sub suction units 121B suction the infusion container 503 together with the main suction unit 121A in accordance with the size of the infusion container 503 in the up-down direction. Accordingly, at least one of the two sub suction units 121B does not suction the infusion container 503 depending on the size of the infusion container 503, in some cases.

The type (size) of the infusion container 503 and the suction unit 121 to be used to suction the infusion container 503 are determined in advance, and information associating the type (size) of the infusion container 503 and the suction unit 121 is stored in the storage unit 200. Accordingly, the second conveyance control unit 145 can identify the suction unit 121 to be used to suction the infusion container 503, by identifying the type of the infusion container 503 to be used for drug-mixing based on the preparation/administration data. Further, the positional relationship between the place at which the infusion container 503 is to be suctioned (for example, the infusion shelf 30 and the drug-mixing unit 40) and the second conveyance unit 120 is determined in advance so that the main suction unit 121A is always used at the time when the second conveyance unit 120 holds the infusion container 503.

The third reading unit 122 reads information included in a first label attached to the body 5031 of the infusion container 503. When the information included in the first label is included in a barcode, the third reading unit 122 may be achieved by a barcode reader.

### <Modification Example 1 of Second Conveyance Unit>

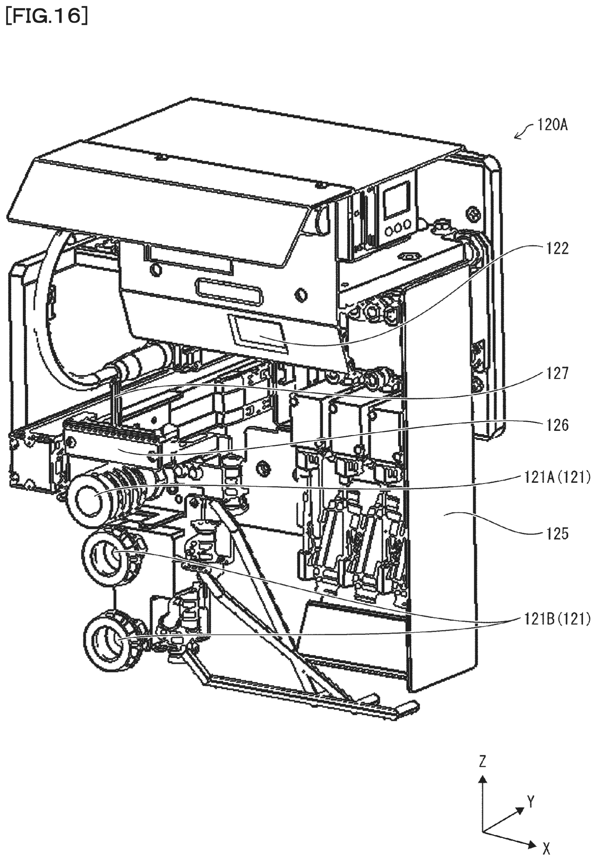

FIG. 16 is a perspective view for illustrating an example of a second conveyance unit 120A that is a modification example of the second conveyance unit 120. As illustrated in FIG. 16, the second conveyance unit 120A includes a transparent plate 126 and a support part 127. Further, a base 125 to which the main suction unit 121A and the sub suction units 121B are attached is movable in the front-rear direction (±Y-axis direction) under the control of the second conveyance control unit 145. FIG. 16 shows a state in which the base 125 has moved forward.

The transparent plate 126 is a member that presses the infusion container 503 to be held by the second conveyance unit 120A. The transparent plate 126 is provided above the main suction unit 121A. Under a state in which the base 125 is moving forward, the third reading unit 122 reads the information of the first label attached to the infusion container 503 via the transparent plate 126.

When the body 5031 of the infusion container 503 is made of a material that is easily deformed, wrinkles are liable to be formed on the body 5031. In particular, wrinkles are liable to be formed on the upper side region of the body 5031. When the first label is attached to the upper side region of the body 5031, there is a possibility that the first label is brought into a distorted state, resulting in that the third reading unit 122 cannot read the information of the first label.

At the time of holding the infusion container 503 having the easily deformable body 5031, the second conveyance control unit 145 adjusts the height of the second conveyance unit 120A so that the transparent plate 126 faces the attaching position of the first label on the body 5031, at which the first label is attached. Accordingly, the second conveyance unit 120A can hold the infusion container 503 under a state in which the attaching position of the first label faces the transparent plate 126. Information as to whether the infusion container 503 is an infusion container 503 for which the information of the first label is read via the transparent plate 126 is stored in the storage unit 200. Further, the attaching position of the first label may be identified, for example, by analyzing an image of the infusion container 503 captured by an image pickup unit included in the second conveyance unit 120A.

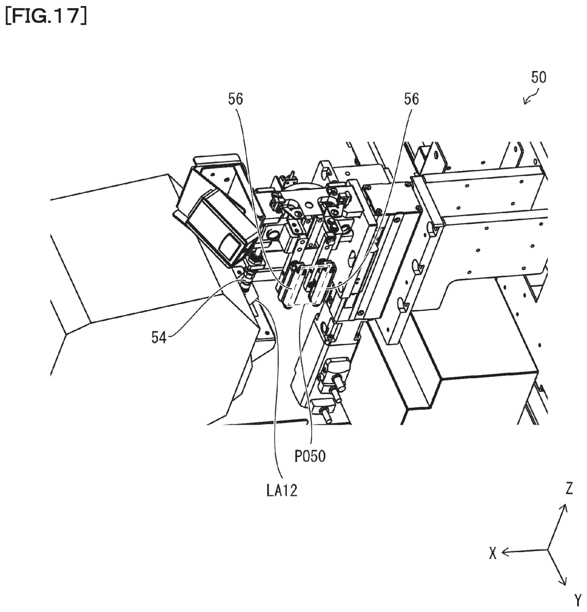

Here, as illustrated in FIG. 17, the printing/inspection unit 50 includes an attaching unit 54 and a guide unit 56. FIG. 17 is a perspective view for illustrating a part of the printing/inspection unit 50. Under a state in which the attaching unit 54 has suctioned a second label LA12 or an unsuitability information label and has moved to an attaching position PO50, the second conveyance unit 120A presses the infusion container 503 after drug injection, against the guide unit 56 at the attaching position PO50. As a result, the second label LA12 or the unsuitability information label is attached to the infusion container 503 after drug injection.

When the third reading unit 122 reads the information of the first label attached to the easily deformable infusion container 503, the second conveyance control unit 145 moves the base 125 forward, and presses the infusion container 503 held by the second conveyance unit 120A against the guide unit 56 of the printing/inspection unit 50. As a result, the attaching position of the first label sandwiched between the transparent plate 126 and the guide unit 56 can be made flat.

Under this state, the third reading unit 122 reads the information of the first label via the transparent plate 126. The attaching position of the first label is in a flat state, and hence the third reading unit 122 can read the information of the first label. The guide unit 56 is an example of a target against which the infusion container 503 is to be pressed, and as long as the attaching position of the first label can be made flat, the second conveyance unit 120A may press the infusion container 503 against any position of the drug-mixing apparatus 1.

The support part 127 is a member that supports a neck portion 5033 (see FIG. 15) of the infusion container 503 held by the second conveyance unit 120A. With the provision of the support part 127, the posture of the neck portion 5033 of the infusion container 503 can be stabilized. Accordingly, a member that holds the neck portion 5033 of the infusion container 503, such as an infusion container holding unit 43 (see FIG. 18) described later, can easily receive the infusion container 503 from the second conveyance unit 120A.

As illustrated in FIG. 16, in the at least one embodiment, the support part 127 is a member having a rod shape. The third reading unit 122 reads the information of the first label without intermediation of the transparent plate 126 depending on the infusion container 503, such as an infusion container 503 that is not easily deformed. In the case of performing such reading, the base 125 has not moved forward, and hence, when the support part 127 has no rod shape, there is a possibility that the support part 127 is included in a range in which the third reading unit 122 reads the information. With the support part 127 having a rod shape, the information of the first label can be easily read by the third reading unit 122.

When the support part 127 has a rod shape, a surface of the support part 127 on the side facing the neck portion 5033 of the infusion container 503 may be flat. In this case, the posture of the neck portion 5033 can be easily stabilized. For example, the support part 127 may have a rectangular parallelepiped shape.

### <Modification Example 2 of Second Conveyance Unit>

In the at least one embodiment, the second conveyance unit 120 includes three suction units 121 (one equipment holding unit) as an equipment holding unit that holds one infusion container 503. The configuration is not limited thereto, and the second conveyance unit 120 may include a plurality of equipment holding units that hold a plurality of infusion containers 503, respectively.

### [Specific Configuration of Drug-mixing Unit]

FIG. 18 is a perspective view for illustrating an example of the configuration of the drug-mixing unit 40. As illustrated in FIG. 18, the drug-mixing unit 40 includes a syringe holding unit 41, a vial holding unit 42, and an infusion container holding unit 43.

The syringe holding unit 41 is a member that holds the syringe 501 conveyed by the first conveyance unit 110. The syringe holding unit 41 includes moving parts 411, a position fixing part 412, a first barrel holding unit 416, and a plunger clamping unit 417.

The moving parts 411 are a pair of moving parts that move between positions close to each other and positions separated from each other. The moving parts 411 clamp the needle 5014 when being located at the positions close to each other. The moving parts 411 clamp the needle 5014 to position the tip of the needle 5014 at a fixed position. Accordingly, during the drug-mixing operation, even when the needle 5014 is bent, puncture with the needle 5014 can be performed at a desired position (correct position) of a stopper provided on the infusion container 503.

The first barrel holding unit 416 is a member that first holds the syringe 501 conveyed from the syringe shelf 10 by the first conveyance unit 110. The first barrel holding unit 416 clamps the side surface of the barrel 5011 (for example, the lower part of the barrel 5011) inserted into the first barrel holding unit 416.

The position fixing part 412 is a member that fixes the position of the syringe 501 in the extending direction of the syringe 501 at the needle-side end portion 5012 and the flange 5013. In the at least one embodiment, the relative position between a second barrel holding unit 413 and a third barrel holding unit 414 that form the position fixing part 412 can be changed along the extending direction of the syringe 501. The second barrel holding unit 413 and the third barrel holding unit 414 are attached so that at least one of the second barrel holding unit 413 or the third barrel holding unit 414 is movable along the extending direction of the syringe 501.

In the at least one embodiment, when the syringe 501 is to be held by the position fixing part 412, the second barrel holding unit 413 abuts against the needle-side end portion 5012 from below, and the third barrel holding unit 414 abuts against the flange 5013 from above. Accordingly, the position fixing part 412 can clamp the syringe 501 along the extending direction of the syringe 501.

The plunger clamping unit 417 is a member that clamps the plunger 5015. In the at least one embodiment, the plunger clamping unit 417 clamps a distal end portion of the plunger 5015. The plunger clamping unit 417 moves in the up-down direction with respect to the moving parts 411, the position fixing part 412, and the first barrel holding unit 416, and thus the plunger 5015 can be moved with respect to the barrel 5011.

The vial holding unit 42 is a member that holds the vial 502 (vial 502 conveyed by the first conveyance unit 110) that houses a drug to be injected into the infusion container 503. The vial holding unit 42 is also a member that holds the vial 502 that houses an infusion having a powdered drug dissolved therein, which is to be injected into the infusion container 503. In the at least one embodiment, the vial holding unit 42 clamps the neck portion of the vial 502.

The infusion container holding unit 43 is a member that holds the infusion container 503 (infusion container 503 conveyed by the second conveyance unit 120). In the at least one embodiment, the infusion container holding unit 43 includes an infusion clamping unit 431 that clamps the neck portion of the infusion container 503, and a mounting table 432 on which the infusion container 503 is placed at the time of withdrawing an infusion from the infusion container 503 or at the time of injecting an infusion into the infusion container 503.

Further, the drug-mixing apparatus 1 may include an image pickup unit that captures an image of the infusion clamping unit 431. The image pickup unit captures an image of the infusion clamping unit 431 at the time when the second conveyance unit 120 delivers the infusion container 503 to the infusion clamping unit 431. After adjusting the positional relationship between the neck portion 5033 of the infusion container 503 and the infusion clamping unit 431 by analyzing the image captured by the image pickup unit, the second conveyance control unit 145 delivers the infusion container 503 held by the second conveyance unit 120 to the infusion clamping unit 431.

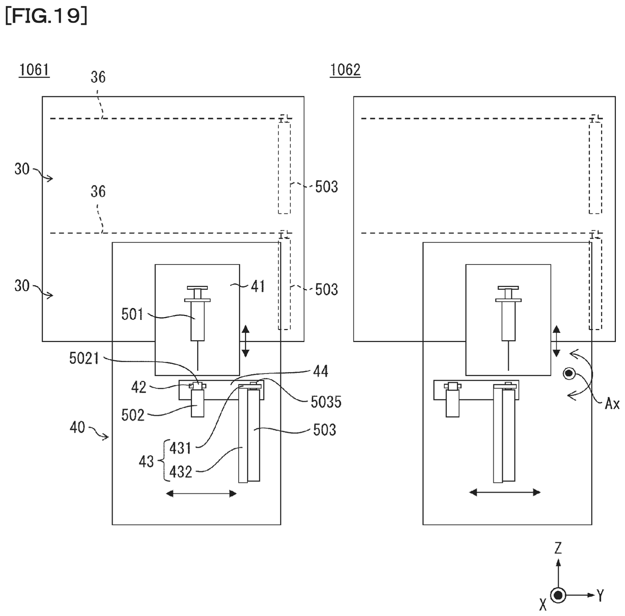

FIG. 19 is a front view for illustrating an example of a schematic configuration of the drug-mixing unit 40, and is an explanatory view for illustrating an operation example of the drug-mixing unit 40. As illustrated in FIG. 18 and FIG. 19, in the at least one embodiment, the drug-mixing unit 40 is provided on a side wall of the infusion shelf 30. Specifically, in the drug-mixing apparatus 1, the drug-mixing unit 40 is arranged so that the syringe holding unit 41, the vial holding unit 42, and the infusion container holding unit 43 face the direction of the syringe shelf 10 and the vial shelf 20 (+X-axis direction).

Further, as illustrated in FIG. 19, the syringe holding unit 41 is movable in the up-down direction. Accordingly, the vial 502 or the infusion container 503 located directly below the syringe 501 held by the syringe holding unit 41 can be punctured with the needle 5014.

Further, the vial holding unit 42 and the infusion container holding unit 43 are movable in the front-rear direction (±Y-axis direction) of the drug-mixing apparatus 1. In the at least one embodiment, the vial holding unit 42 and the infusion container holding unit 43 are attached to a base 44, and the base 44 moves in the front-rear direction of the drug-mixing apparatus 1. Thus, the vial holding unit 42 and the infusion container holding unit 43 can move between a position indicated by reference numeral 1061 in FIG. 19 and a position indicated by reference numeral 1062 in FIG. 19. The position indicated by reference numeral 1061 in FIG. 19 is a first puncture position for puncturing the vial 502 with the needle 5014, and the position indicated by reference numeral 1062 in FIG. 19 is a second puncture position for puncturing the infusion container 503 with the needle 5014. Accordingly, the stopper 5021 of the vial 502 held by the vial holding unit 42, or a stopper 5035 of the infusion container 503 held by the infusion container holding unit 43 can be punctured with the needle 5014 of the syringe 501 held by the syringe holding unit 41. However, instead of the base 44, the syringe holding unit 41 may move in the front-rear direction of the drug-mixing apparatus 1.

Further, as indicated by reference numeral 1062 in FIG. 19, the drug-mixing unit 40 is provided so as to be rotatable about a rotation axis Ax (with Ax serving as the rotation axis). The rotation axis Ax is an axis extending in a direction perpendicular (±X-axis direction) to a pedestal on which the syringe holding unit 41, the vial holding unit 42, and the infusion container holding unit 43 are provided.

At the first puncture position indicated by reference numeral 1061 in FIG. 19, the drug-mixing unit 40 rotates about the rotation axis Ax, and thus the vial 502 can be moved to a position above the syringe 501. At the second puncture position indicated by reference numeral 1062 in FIG. 19, the drug-mixing unit 40 rotates about the rotation axis Ax, and thus the infusion container 503 can be moved to a position above the syringe 501. Under those states, the drug-mixing unit 40 can move the plunger 5015 to transfer liquid between the syringe 501 and the vial 502 or the infusion container 503.

### <Example of Drug-mixing Processing for Powdered Drug>

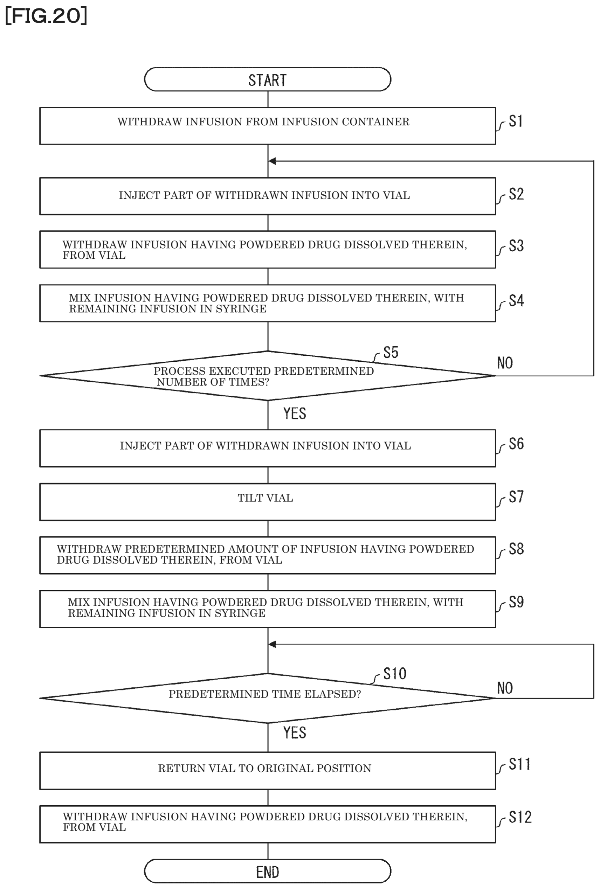

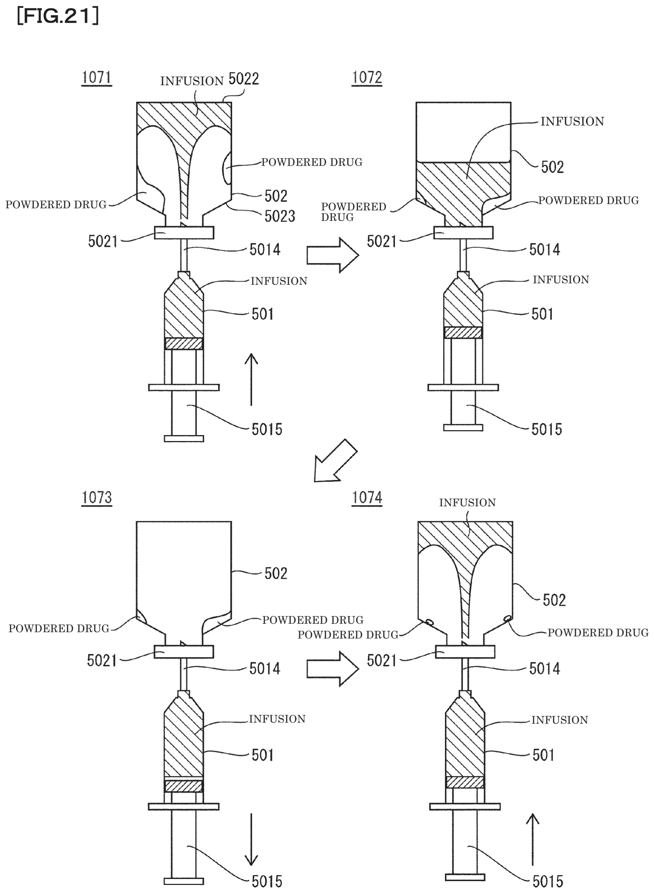

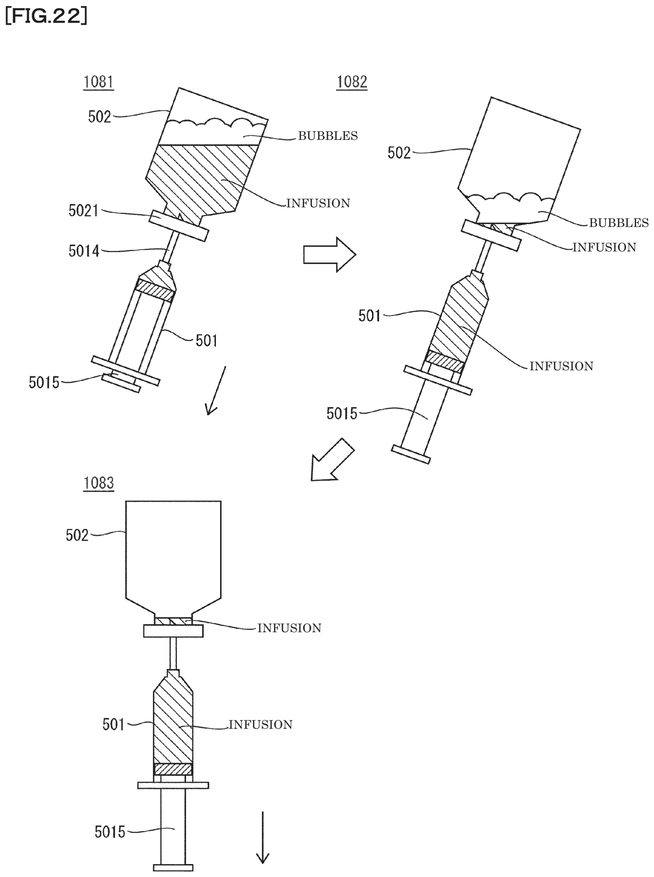

FIG. 20 is a flowchart for illustrating an example of a process flow at the time of mixing a powdered drug housed in the vial 502, into an infusion. As a premise of this process, the syringe 501, the vial 502, and the infusion container 503 to be used for drug-mixing are held by the drug-mixing unit 40. FIG. 21 is a schematic view for illustrating an operation example of the drug-mixing unit 40 at the time of dissolving a powdered drug in an infusion. FIG. 22 is a schematic view for illustrating an operation example of the drug-mixing unit 40 at the time of withdrawing an infusion having a powdered drug dissolved therein, from the vial 502.

As illustrated in FIG. 20, a drug-mixing-unit control unit 144 controls the syringe holding unit 41 to puncture the stopper 5035 of the infusion container 503 with the needle 5014 of the syringe 501, and then withdraws the infusion from the infusion container 503 (Step S1). The amount of the infusion withdrawn from the infusion container 503 by the drug-mixing-unit control unit 144 is the withdrawal amount of the infusion indicated in the above-mentioned preparation/administration data. This withdrawal amount may be set to be larger than a specified amount. For example, when the specified amount is 5 mL, the drug-mixing-unit control unit 144 withdraws, for example, 10 mL of the infusion from the infusion container 503.

The specified amount is an amount of infusion that can be injected into the vial 502, and is determined for each type of vial 502 in which a powdered drug has been housed. When an infusion is injected into the vial 502, the internal pressure of the vial 502 increases. When the internal pressure continues to increase, there is a risk that a crack may occur in the stopper 5021, resulting in that the infusion may spurt out from the stopper 5021. The specified amount may be determined, for example, through experiments, to be such an amount that a crack does not occur even without creating a negative pressure by withdrawing the infusion or gas from the vial 502. The type of vial 502 in which a powdered drug has been housed and the specified amount of infusion that can be injected into the vial 502 are stored in the storage unit 200 in association with each other. However, the specified amount may be uniformly determined for all vials 502. The information indicating the type of the vial 502 described above is included in the preparation/administration data.

After that, the drug-mixing-unit control unit 144 withdraws the needle 5014 of the syringe 501 from the stopper 5035, moves the base 44, and then punctures the stopper 5021 with the needle 5014 attached to the syringe 501. After that, the drug-mixing-unit control unit 144 rotates the drug-mixing unit 40 about the axis Ax until the vial 502 reaches a position directly above the syringe 501.

The drug-mixing-unit control unit 144 controls the plunger clamping unit 417 to move the plunger 5015, to thereby inject part of the infusion housed in the syringe 501, into the vial 502 in which the powdered drug has been housed (Step S2). With this injection, the powdered drug can be dissolved in the infusion in the vial 502.

The drug-mixing-unit control unit 144 injects, for example, a specified amount of the infusion (for example, 5 mL out of 10 mL) into the vial 502 as part of the infusion housed in the syringe 501. Information indicating the injection amount into the vial 502 is stored in the storage unit 200.

In the at least one embodiment, as indicated by reference numeral 1071 in FIG. 21, the plunger clamping unit 417 injects (sprays) the infusion housed in the syringe 501 into the vial 502 under a state in which the stopper 5021 has been punctured with the needle 5014 attached to the syringe 501 and having the tip portion pointing upward. Accordingly, the infusion injected into the vial 502 easily spreads throughout the entire vial 502. Thus, the powdered drug can be efficiently dissolved in the infusion.

Next, the drug-mixing-unit control unit 144 controls the plunger clamping unit 417 to move the plunger 5015, to thereby withdraw the infusion having the powdered drug dissolved therein, from the vial 502 into which the infusion has been injected in Step S2, using the same syringe 501 that has injected the infusion in Step S2 (Step S3). With the above-mentioned withdrawal, the infusion having the powdered drug dissolved therein, which has been withdrawn in Step S3, is mixed with the remaining infusion in the syringe 501, which has not been injected into the vial 502.

Reference numeral 1072 in FIG. 21 indicates a state in which the injection of the infusion into the vial 502 by the plunger clamping unit 417 has been completed. While maintaining this state, the drug-mixing-unit control unit 144 draws the infusion having the powdered drug dissolved therein, which has been housed in the vial 502, into the syringe 501, as indicated by reference numeral 1073 in FIG. 21. As described above, when the syringe 501 having the remaining infusion housed therein is used to withdraw the infusion having the powdered drug dissolved therein, from the vial 502 into which the infusion has been injected in Step S2, the remaining infusion and the infusion having the powdered drug dissolved therein can be mixed inside the syringe 501.

The drug-mixing-unit control unit 144 determines whether the process steps of Step S2 to Step S4 have been executed a predetermined number of times (Step S5). The predetermined number of times is a value indicating a preset specified number of times minus one. Information indicating the specified number of times is stored in the storage unit 200. The specified number of times may be a constant value regardless of the type of powdered drug, or may be a value set for each type of powdered drug. The specified number of times is set to a value of 2 or more. The specified number of times may be determined, for example, through experiments, to be the number of times that the powdered drug housed in the vial 502 is sufficiently dissolved in the infusion.

When the drug-mixing-unit control unit 144 determines that the process steps of Step S2 to Step S4 have not been executed the predetermined number of times (NO in Step S5), the process returns to the process step of Step S2 and executes the process steps of Step S2 to Step S4 until the predetermined number of times is reached. In the second round or the subsequent rounds of the process step of Step S2, the drug-mixing unit 40 (plunger clamping unit 417) injects the infusion having the powdered drug dissolved therein (part of the infusion mixed in Step S4) into the vial 502 as part of the infusion to be injected into the vial 502. As indicated by reference numeral 1073 and reference numeral 1074 in FIG. 21, the drug-mixing-unit control unit 144 injects the infusion having the powdered drug dissolved therein, which has been drawn into the syringe 501, into the vial 502, while maintaining a state in which the stopper 5021 has been punctured with the needle 5014 of the syringe 501. Further, in the second round or the subsequent rounds of the process step of Step S4, the remaining infusion in the syringe 501 is the infusion having the powdered drug dissolved therein. Further, the injection amount of the infusion in the second round or the subsequent rounds may be smaller than the specified amount.

When the drug-mixing-unit control unit 144 determines that the process steps of Step S2 to Step S4 have been executed the predetermined number of times (YES in Step S5), the drug-mixing-unit control unit 144 injects the infusion having the powdered drug dissolved therein, which has been withdrawn from the vial 502, into the vial 502 (Step S6).

Next, the drug-mixing-unit control unit 144 rotates the drug-mixing unit 40 to tilt the vial 502 so that the stopper 5021 changes from a first state of facing downward to a second state of facing the horizontal plane side (Step S7). The drug-mixing-unit control unit 144 controls the plunger clamping unit 417 under the second state to withdraw the infusion having the powdered drug dissolved therein, from the vial 502 (Step S8). Accordingly, the infusion having the powdered drug dissolved therein, which has been withdrawn in Step S8, is mixed with the remaining infusion in the syringe 501 (Step S9).

At the time of withdrawing the infusion having the powdered drug dissolved therein, from the vial 502, the vial 502 is tilted so that the vial 502 is brought into the second state on the horizontal plane side rather than the first state. Accordingly, the infusion having the powdered drug dissolved therein, which has adhered to a bottom part 5022 of the vial 502, easily flows toward the stopper 5021 side due to its own weight.

After withdrawing a predetermined amount of the infusion having the powdered drug dissolved therein, from the vial 502 in Step S8, the drug-mixing-unit control unit 144, determines whether a predetermined time has elapsed (Step S10). The drug-mixing-unit control unit 144 waits until the predetermined time has elapsed (in the case of NO in Step S10). When the drug-mixing-unit control unit 144 determines that the predetermined time has elapsed (in the case of YES in Step S10), the drug-mixing-unit control unit 144 controls the plunger clamping unit 417 to withdraw the infusion having the powdered drug dissolved therein, from the vial 502 again (Step S12). Accordingly, a predetermined withdrawal amount or part thereof of the infusion having the powdered drug dissolved therein can be withdrawn from the vial 502. The amount of the infusion having the powdered drug dissolved therein, which is to be finally withdrawn from the vial 502, (referred to as "final withdrawal amount") is stored in the storage unit 200. Such an amount of the infusion may be included in the preparation/administration data.

When an infusion is injected into the vial 502, bubbles are generated depending on the type of powdered drug. In order to withdraw the infusion having the powdered drug dissolved therein, from the vial 502, it is preferred to change the bubbles into a liquid. This is because it is difficult to withdraw the infusion in the state of bubbles.

Under a state in which bubbles are generated as indicated by reference numeral 1081 in FIG. 22, the drug-mixing-unit control unit 144 performs a process of withdrawing a predetermined amount of the infusion having the powdered drug dissolved therein (process step of Step S8 illustrated in FIG. 20), and thus only the infusion can be withdrawn as indicated by reference numeral 1082 in FIG. 22. Then, only the infusion is withdrawn from the vial 502, and thus the inside of the vial 502 can be brought into a negative pressure state. With this negative pressure, as indicated by reference numeral 1083 in FIG. 22, the bubbles generated due to the injection of the infusion can be eliminated and changed into a liquid. Accordingly, the infusion that has changed from bubbles to a liquid can be withdrawn more quickly than in the case of waiting for the bubbles to disappear naturally and then withdrawing the infusion.

The predetermined amount is set to an amount smaller than the amount of the infusion injected into the vial 502. This is because an amount corresponding to part of the infusion, which has become bubbles, is prevented from being withdrawn. Further, information indicating the predetermined amount and information indicating the predetermined time are stored in the storage unit 200. The predetermined amount and the predetermined time may be constant values regardless of the type of powdered drug, or may be values set for each type of powdered drug. Further, the predetermined amount may be determined, for example, through experiments, in order to consider the proportion of the injected infusion that becomes bubbles. The predetermined time may be determined, for example, through experiments, in order to consider the time taken for bubbles to change into a liquid.

The predetermined time may be different depending on a method of withdrawing the infusion having the powdered drug dissolved therein from the vial 502. Examples of the method of withdrawing the infusion include two types of methods, that is, partial withdrawal and whole withdrawal. The partial withdrawal means withdrawing part of the infusion having the powdered drug dissolved therein, which has been housed in the vial 502, from the vial 502. The whole withdrawal means withdrawing the whole amount of the infusion having the powdered drug dissolved therein, which has been housed in the vial 502, from the vial 502.

Regardless of whether the partial withdrawal or the whole withdrawal, the amount of the powdered drug contained in part or all of the infusion having the powdered drug dissolved therein, which is to be withdrawn from the vial 502 in Step S12, is the prescribed amount (amount indicated in the prescription). Through the process steps up to Step S12, the powdered drug is substantially uniformly dissolved in the infusion. Accordingly, even with the partial withdrawal, the prescribed amount of the powdered drug can be withdrawn in the process step of Step S12.

Further, in the case of the partial withdrawal, the drug-mixing-unit control unit 144 may withdraw the infusion having the powdered drug dissolved therein from the vial 502, by an amount slightly larger than the final withdrawal amount. In the operation of withdrawing the infusion, there is a possibility that gas in the vial 502 enters the syringe 501, resulting in that the amount of the infusion withdrawn into the syringe 501 becomes smaller by the amount of the gas that has entered the syringe 501. In order to reduce such a possibility, in Step S12, the drug-mixing-unit control unit 144 may withdraw the infusion having the powdered drug dissolved therein, from the vial 502 by an amount slightly larger than the final withdrawal amount, and then may move the plunger 5015 to the position of the scale indicating the final withdrawal amount by the amount withdrawn in excess.

At this time, in Step S12, the drug-mixing-unit control unit 144 controls the movement of the plunger 5015 while maintaining the positional relationship between the syringe 501 and the vial 502 (that is, under the state indicated by reference numeral 1083 in FIG. 22). However, without performing this control in Step S12, the drug-mixing-unit control unit 144 may withdraw the infusion having the powdered drug dissolved therein, from the vial 502 by the final withdrawal amount.

When a predetermined time set so as to correspond to the partial withdrawal is referred to as "first predetermined time," a second predetermined time shorter than the first predetermined time may be set as a predetermined time set so as to correspond to the whole withdrawal.

Consideration is made on a case in which, in Step S12, the drug-mixing-unit control unit 144 uses the syringe 501 to withdraw part of the infusion having the powdered drug dissolved therein, from the vial 502. In this case, after executing a dissolving operation of dissolving the powdered drug in the infusion in Step S2 to Step S4 and Step S6, the drug-mixing-unit control unit 144 withdraws the infusion having the powdered drug dissolved therein, from the vial 502 in Step S12 after the first predetermined time has elapsed.

Further, consideration is made on a case in which, in Step S12, the drug-mixing-unit control unit 144 uses the syringe 501 to withdraw the whole amount (all) of the infusion having the powdered drug dissolved therein, from the vial 502. In this case, after executing the dissolving operation of dissolving the powdered drug in the infusion in Step S2 to Step S4 and Step S6, the drug-mixing-unit control unit 144 withdraws the infusion having the powdered drug dissolved therein, from the vial 502 in Step S12 after the second predetermined time has elapsed.

In the at least one embodiment, the first predetermined time and the second predetermined time are set for each of a plurality of types of powdered drugs that can be handled in the drug-mixing apparatus 1. The first predetermined time and the second predetermined time can be set in consideration of the fact that the time taken for bubbles to change into a liquid differs depending on the type of powdered drug. The first predetermined time and the second predetermined time are stored in the storage unit 200 in association with information indicating the type of drug. In Step S10, the drug-mixing-unit control unit 144 identifies the first predetermined time or the second predetermined time corresponding to the powdered drug to be mixed, which is included in the prescription data, and stops the drug-mixing operation for the identified first predetermined time or second predetermined time. The drug-mixing-unit control unit 144 starts clocking the first predetermined time and the second predetermined time, for example, at the time when the process step of Step S6 is completed. However, the clocking of the first predetermined time and the second predetermined time may be started, for example, at the time when the process step of Step S8 is completed.

In the case of the whole withdrawal, even when the stop time is slightly shorter and some bubbles remain, there is no or little influence on the withdrawal accuracy. Accordingly, the drug-mixing apparatus 1 can shorten the time required for the drug-mixing operation while ensuring the withdrawal accuracy, by setting the second predetermined time shorter than the first predetermined time. Each of the first predetermined time and the second predetermined time may be set to an extent that does not affect the withdrawal accuracy. In the case of the partial withdrawal, when bubbles remain, the withdrawal accuracy is affected, and hence, the second predetermined time is set to a time taken until most of the bubbles change into a liquid.

In the at least one embodiment, the injection and withdrawal of the infusion into and from the vial 502 are performed a plurality of times. The withdrawal of the infusion having the prescribed amount of the powdered drug dissolved therein from the vial 502 in Step S12 is the final withdrawal of the infusion from the vial 502. The drug-mixing-unit control unit 144 waits, for the first predetermined time or the second predetermined time, for the bubbles generated due to the injection of the infusion to disappear before the final withdrawal. That is, after executing the dissolving operation, the drug-mixing-unit control unit 144 performs the final withdrawal of the infusion having the powdered drug dissolved therein from the vial 502, after the first predetermined time or the second predetermined time has elapsed.

Even when the injection and withdrawal of the infusion into and from the vial 502 are performed only once, the drug-mixing-unit control unit 144 waits, for the first predetermined time or the second predetermined time, for the bubbles generated due to the injection of the infusion to disappear before the withdrawal. In this case, the first withdrawal of the infusion from the vial 502 (the withdrawal of the infusion performed only once) corresponds to the above-mentioned final withdrawal of the infusion having the powdered drug dissolved therein from the vial 502.

Prior to Step S12, the drug-mixing-unit control unit 144 rotates the drug-mixing unit 40 to tilt the vial 502 so that the stopper 5021 is brought into a third state of facing downward from the second state (Step S11). With the change in the posture of the vial 502 from the second state to the third state, the infusion that has accumulated in a shoulder portion 5023 of the vial 502 under the second state easily flows toward the stopper 5021 side due to its own weight. In the at least one embodiment, the drug-mixing-unit control unit 144 changes the posture of the vial 502 from the second state to the first state. Accordingly, most of the infusion that has accumulated in the shoulder portion 5023 easily flows to the stopper 5021.

### <Example of Drug-mixing Processing during Waiting for Predetermined Time>

The drug-mixing-unit control unit 144 waits for a predetermined time after injecting the infusion into the vial 502, and then withdraws the infusion from the vial 502. Specifically, as described above, in Step S10, the drug-mixing-unit control unit 144 waits, for a predetermined time, for the bubbles generated due to the injection of the infusion to disappear. When this drug-mixing operation is referred to as "first drug-mixing operation for first prescription data," a drug-mixing operation for second prescription data executed after the first drug-mixing operation is referred to as "second drug-mixing operation."

During the waiting for the predetermined time in the first drug-mixing operation, the first conveyance control unit 143 controls the first conveyance unit 110 to remove the syringe 501 and the vial 502 being used in the first drug-mixing operation, from the drug-mixing unit 40. Further, the second conveyance control unit 145 controls the second conveyance unit 120 to remove the infusion container 503 being used in the first drug-mixing operation, from the drug-mixing unit 40. The control unit 140 may start the control of the first conveyance unit 110 and/or the second conveyance unit 120 at the time when clocking of the predetermined time is started.

Next, the first conveyance control unit 143 controls the first conveyance unit 110 to take out the syringe 501 and the vial 502 to be used for the second drug-mixing operation, from the syringe shelf 10 and the vial shelf 20, respectively, and cause the syringe holding unit 41 and the vial holding unit 42 of the drug-mixing unit 40 to hold the syringe 501 and the vial 502, respectively. After the needle cap 5016 is removed from the syringe 501 by the cap removal unit 180, the syringe 501 is conveyed to the drug-mixing unit 40. Further, the second conveyance control unit 145 controls the second conveyance unit 120 to take out the infusion container 503 to be used for the second drug-mixing operation, from the infusion shelf 30, and cause the infusion container holding unit 43 of the drug-mixing unit 40 to hold the infusion container 503. After that, the drug-mixing-unit control unit 144 starts the second drug-mixing operation by the drug-mixing unit 40.

Accordingly, the drug-mixing apparatus 1 can execute the second drug-mixing operation during the waiting for the predetermined time in the first drug-mixing operation. Accordingly, the drug-mixing apparatus 1 can shorten the time required for the drug-mixing operation.

In the at least one embodiment, the first conveyance unit 110 includes the first claw part 111 and the second claw part 112. For example, the first conveyance unit 110 holds the syringe 501 being used in the first drug-mixing operation by the first claw part 111, and conveys the syringe 501 to a standby space for temporarily placing the syringe 501 on standby. The standby space may be provided with a locking part that locks the syringe 501. After placing the syringe 501 on standby in the standby space, the first conveyance unit 110 holds the vial 502 being used in the first drug-mixing operation by the first claw part 111.

However, when the control unit 140 determines, based on the prescription data, that the same powdered drug is used in the first drug-mixing operation and the second drug-mixing operation, and that no infusion remains in the syringe 501 being used in the first drug-mixing operation, the control unit 140 can perform the subsequent process. The control unit 140 may keep the syringe 501 held by the first conveyance unit 110, in order to use the syringe 501 for the second drug-mixing operation.

Further, in the at least one embodiment, after causing the suction unit 121 to suction the infusion container 503 being used in the first drug-mixing operation, the second conveyance unit 120 conveys the infusion container 503 to a standby space for placing the infusion container 503 on standby. The standby space may be provided with a locking part that locks the infusion container 503.

After the second drug-mixing operation by the drug-mixing unit 40 is completed, the first conveyance control unit 143 controls the first conveyance unit 110 to remove the syringe 501 and the vial 502 used in the second drug-mixing operation and discard the syringe 501 and the vial 502 into the waste box 70. After that, the first conveyance control unit 143 delivers the syringe 501 and the vial 502 being used in the first drug-mixing operation, which have been removed from the drug-mixing unit 40, to the drug-mixing unit 40. In the at least one embodiment, the first conveyance unit 110 delivers the vial 502 held by the first conveyance unit 110, to the vial holding unit 42. After that, the first conveyance unit 110 holds the syringe 501 that has been placed on standby in the standby space, and delivers the syringe 501 to the syringe holding unit 41.

Further, after the second drug-mixing operation by the drug-mixing unit 40 is completed, the second conveyance control unit 145 controls the second conveyance unit 120 to remove the infusion container 503 used in the second drug-mixing operation and convey the infusion container 503 to the printing/inspection unit 50. After that, the second conveyance control unit 145 delivers the infusion container 503 being used in the first drug-mixing operation, which has been removed from the drug-mixing unit 40, to the drug-mixing unit 40. In the at least one embodiment, the second conveyance unit 120 holds the infusion container 503 that has been placed on standby in the standby space, and delivers the infusion container 503 to the infusion container holding unit 43. After the syringe 501, the vial 502, and the infusion container 503 being used in the first drug-mixing operation are delivered to the drug-mixing unit 40, the drug-mixing-unit control unit 144 resumes the first drug-mixing operation when the predetermined time has elapsed.

As described above, the first conveyance unit 110 holds the vial 502 until the first drug-mixing operation is resumed. Accordingly, it is not required to provide a standby space for placing the vial 502 on standby, in the drug-mixing apparatus 1. For example, consideration is made on a case in which the first conveyance unit 110 holds the syringe 501 instead of the vial 502. In this case, until the first drug-mixing operation is resumed, the first conveyance unit 110 places the vial 502 on standby in the standby space, and holds the syringe 501. Accordingly, in this case, it is not required to provide a standby space for placing the syringe 501 on standby, in the drug-mixing apparatus 1.

The delivery of the syringe 501, the vial 502, and the infusion container 503 being used in the first drug-mixing operation to the drug-mixing unit 40 may be performed immediately after the completion of the second drug-mixing operation (immediately after the procedure executed in the second drug-mixing operation is completed). Alternatively, such delivery may be performed, for example, after the second conveyance unit 120 has delivered the infusion container 503 given after the second drug-mixing operation, to the infusion receiving shelf 60.

The drug-mixing-unit control unit 144 may continue the second drug-mixing operation even when the predetermined time has elapsed during the execution of the second drug-mixing operation. In this case, the drug-mixing-unit control unit 144 resumes the first drug-mixing operation after the second drug-mixing operation is completed, thereby being capable of reducing the possibility that the control in the case of performing the first drug-mixing operation and the second drug-mixing operation in parallel becomes complicated.

Consideration is made on a case in which the first conveyance unit 110 includes at least one claw part in addition to the first claw part 111 and the second claw part 112. In this case, the first conveyance control unit 143 may cause one claw part to hold the syringe 501 being used in the first drug-mixing operation, and cause another claw part to hold the vial 502 being used in the first drug-mixing operation.

As described above, part of the plurality of claw parts holds at least one of the syringe 501 or the vial 502 being used in the first drug-mixing operation until the first drug-mixing operation is resumed. When the first conveyance unit 110 holds both the syringe 501 and the vial 502 being used in the first drug-mixing operation, until the first drug-mixing operation is resumed, it is not required to provide each of a standby space for placing the syringe 501 on standby and a standby space for placing the vial 502 on standby, in the drug-mixing apparatus 1.

When the second conveyance unit 120 includes a plurality of suction unit groups that hold a plurality of infusion containers 503, respectively, the second conveyance control unit 145 may cause part of the suction unit groups to hold the infusion container 503 being used in the first drug-mixing operation, until the first drug-mixing operation is resumed. In this case, it is not required to provide a standby space for placing the infusion container 503 on standby, in the drug-mixing apparatus 1.

Consideration is made on a case in which the equipment removal unit (the first conveyance unit 110, the second conveyance unit 120) includes a plurality of equipment holding units as described above. In this case, part of the plurality of equipment holding units may hold at least one of the syringe 501, the vial 502, or the infusion container 503 being used in the first drug-mixing operation, until the drug-mixing unit 40 resumes the first drug-mixing operation.

In this case, the equipment removal unit holds the removed equipment as it is, and thus it is not required to provide a standby space for placing the equipment held by the equipment removal unit on standby until the first drug-mixing operation is resumed. Further, the drug-mixing apparatus 1 can cause the equipment holding unit that is not holding the equipment removed by the equipment removal unit, to hold the equipment for the second drug-mixing operation. Accordingly, the equipment removal unit can hold the equipment for the first drug-mixing operation, and can also hold the equipment for the second drug-mixing operation. Accordingly, the equipment removal unit can be used in common for the first drug-mixing operation and the second drug-mixing operation.

In the above-mentioned example, the control unit 140 starts the second drug-mixing operation during the waiting for the predetermined time in the first drug-mixing operation in Step S10. The configuration is not limited thereto, and the drug-mixing-unit control unit 144 may wait, for a predetermined time, for the powdered drug to dissolve in the infusion, for example, after the process step of Step S2. The control unit 140 may start the second drug-mixing operation during the waiting for the predetermined time for this dissolution (stirring) in the first drug-mixing operation. In this case, the drug-mixing-unit control unit 144 starts clocking the predetermined time, for example, at the time when the process step of Step S2 is completed.

However, the drug-mixing-unit control unit 144 may wait, for a predetermined time, for the powdered drug to dissolve in the infusion, for example, after the process step of Step S3. In this case, the drug-mixing-unit control unit 144 starts clocking the predetermined time, for example, at the time when the process step of Step S3 is completed. Further, the predetermined time for waiting for the bubbles generated due to the injection of the infusion to disappear may be the predetermined time for waiting for the powdered drug to dissolve in the infusion.

### (Example of Drug-mixing Processing in Case of Plurality of Pieces of Prescription Data being Grouped)

Consideration is made on a case in which a plurality of pieces of prescription data are grouped in the prescription data. In this case, for example, when the control unit 140 stops the first drug-mixing operation in Step S10, the control unit 140 determines whether the first prescription data and the second prescription data belong to the same group.

Consideration is made on a case in which the control unit 140 determines that the first prescription data and the second prescription data belong to the same group. In this case, as described above, the first conveyance control unit 143 and the second conveyance control unit 145 remove the syringe 501, the vial 502, and the infusion container 503 being used in the first drug-mixing operation, from the drug-mixing unit 40. That is, the first conveyance unit 110 and the second conveyance unit 120 remove the syringe 501, the vial 502, and the infusion container 503 in the middle of the first drug-mixing operation, and thus the drug-mixing unit 40 is brought into a state of being capable of starting the second drug-mixing operation.

Meanwhile, consideration is made on a case in which the control unit 140 determines that the first prescription data and the second prescription data belong to different groups. In this case, the first conveyance control unit 143 controls the first conveyance unit 110 to remove the syringe 501 and the vial 502 used in the first drug-mixing operation, from the drug-mixing unit 40 after the first drug-mixing operation is completed. Further, the second conveyance control unit 145 controls the second conveyance unit 120 to remove the infusion container 503 used in the first drug-mixing operation, from the drug-mixing unit 40 after the first drug-mixing operation is completed. That is, the drug-mixing unit 40 is not brought into a state of being capable of starting the second drug-mixing operation, until the first drug-mixing operation is completed.

For example, consideration is made on a case in which a plurality of pieces of prescription data are grouped into a group for a ward A and a group for a ward B. In this case, the control unit 140 controls each part of the drug-mixing apparatus 1 to continuously execute a drug-mixing operation (preparation) for a plurality of patients hospitalized in the ward A. In the continuous execution of the drug-mixing operation, the control unit 140 may start the second drug-mixing operation in the middle of the first drug-mixing operation, as described above. When executing the drug-mixing operation for the last patient among the plurality of patients hospitalized in the ward A, the control unit 140 does not execute the drug-mixing operation for a patient hospitalized in the ward B until the drug-mixing operation for the last patient is completed.

In this case, when a plurality of pieces of prescription data are grouped, the drug-mixing apparatus 1 can complete the drug-mixing operation for each group. Accordingly, the infusion container 503 after drug-mixing is easily managed by a user.

### (Change in Order of Drug-mixing Operation)

The control unit 140 may change the order of the drug-mixing operation for each of the plurality of pieces of prescription data. For example, the control unit 140 may change the order of the drug-mixing operation so that a drug-mixing operation for a powdered drug and a drug-mixing operation for a liquid drug can be executed alternately. When executing a drug-mixing operation for a powdered drug as the first drug-mixing operation, the control unit 140 starts a drug-mixing operation for a liquid drug as the second drug-mixing operation during the waiting for the predetermined time in the first drug-mixing operation. In this case, the control unit 140 can execute a drug-mixing operation for a liquid drug that does not require the process as illustrated in FIG. 20 (process for dissolving and stirring a powdered drug in an infusion), while the bubbles are being eliminated in the drug-mixing operation for a powdered drug. The time required for the drug-mixing operation for a liquid drug is shorter than the time required for the drug-mixing operation for a powdered drug, resulting in that the time required for the drug-mixing operation for the entirety of the plurality of pieces of prescription data can be shortened.

Further, information indicating the time required for each drug-mixing operation may be stored in the storage unit 200. In this case, the control unit 140 may change the order of the drug-mixing operation by referring to the information so that the time required for the drug-mixing operation for the entirety of the plurality of pieces of prescription data becomes the shortest.

### (Resumption Timing of First Drug-mixing Operation)

The resumption timing of the first drug-mixing operation is not always required to fall after the completion of the second drug-mixing operation, and may fall after the completion of a predetermined procedure of the second drug-mixing operation. For example, when executing a drug-mixing operation for a powdered drug as the second drug-mixing operation, the control unit 140 may resume the first drug-mixing operation during the waiting for the predetermined time in the middle of the second drug-mixing operation.

### <Modification Examples>

In Step S5, the drug-mixing-unit control unit 144 may determine whether the process steps of Step S2 to Step S4 have been executed a specified number of times. When the process steps of Step S2 to Step S4 have been executed the specified number of times, the drug-mixing-unit control unit 144 may advance the process to the process step of Step S12. Further, the drug-mixing-unit control unit 144 may perform the process steps of Step S2 to Step S4 only once.

The drug-mixing-unit control unit 144 is not always required to perform the process of tilting the vial 502 (the process steps of Step S7 and Step S11). Further, the drug-mixing-unit control unit 144 is not always required to perform the process of withdrawing the remaining infusion from the vial 502 after a predetermined time has elapsed after injecting a predetermined amount of the infusion into the vial 502 (the process steps of Step S8, Step S10, and Step S12). The drug-mixing-unit control unit 144 may perform the process steps of Step S8, Step S10, and Step S12, for example, only for a powdered drug that is liable to cause foaming at the time when the infusion is injected into the vial 502. Further, the drug-mixing-unit control unit 144 may perform the process step of Step S8, for example, only for a powdered drug for which it takes time for bubbles to disappear. Information indicating whether or not the process steps of Step S8, Step S10, and/or Step S12 is required to be performed may be stored in the storage unit 200 in association with information indicating the type of drug.

Further, even when the drug-mixing-unit control unit 144 does not perform the process step of Step S8, the drug-mixing-unit control unit 144 may perform the process step of Step S10. Also in this case, as described above, the time taken until the infusion having the powdered drug dissolved therein is withdrawn from the vial 502 in Step S12 after executing the dissolving operation of dissolving the powdered drug in the infusion in Step S2 to Step S4, or Step S2 to Step S4 and Step S6 may be made different depending on the partial withdrawal and the whole withdrawal.

### [Control during Power Off]

The control unit 140 stores the type of the syringe 501 or the vial 502 being conveyed by the first conveyance unit 110, in a volatile memory. Accordingly, when the power of the drug-mixing apparatus 1 is turned off due to a power failure or the like while the first conveyance unit 110 is conveying the syringe 501 or the vial 502, there is a risk that the control unit 140 may not be able to manage whether the equipment held by the first conveyance unit 110 is a syringe 501 or a vial 502. Thus, after power is restored, the control unit 140 removes the equipment, which has been held by the first conveyance unit 110 when the power has been turned off, from the first conveyance unit 110, and enables redoing the drug-mixing operation.

The drug-mixing apparatus 1 may include, for example, a camera that captures images of the syringe 501 and the vial 502 being conveyed by the first conveyance unit 110. After power is restored, the control unit 140 may determine whether the equipment being conveyed by the first conveyance unit 110 is a syringe 501 or a vial 502, based on an image captured by the camera while the drug-mixing apparatus 1 is operating. As for the syringe 501, the control unit 140 may determine whether the needle cap 5016 is attached to the needle 5014 of the syringe 501 being conveyed by the first conveyance unit 110.

The control unit 140 determines whether the equipment being conveyed is a syringe 501 or a vial 502, for example, by comparing a silhouette image of the equipment shown in the image with a silhouette image of the syringe 501 and a silhouette image of the vial 502 stored in the storage unit 200. Further, the control unit 140 compares, for example, a silhouette image of the equipment shown in the image with an image of the syringe 501 having the needle cap 5016 attached thereto and an image of the syringe 501 having no needle cap 5016 attached thereto, which are stored in the storage unit 200. Accordingly, the control unit 140 determines whether the needle cap 5016 is attached to the needle 5014 of the syringe 501 being conveyed.

As long as the equipment being conveyed by the first conveyance unit 110 is detected so that the control unit 140 can perform the above-mentioned determination, the member that detects the equipment being conveyed may be a member other than a camera.

### <Case of Including Needle Removal Unit>

As described above, the drug-mixing apparatus 1 includes the needle removal unit 170. Accordingly, after power is restored, when the control unit 140 determines that the equipment being conveyed is a syringe 501 having no needle cap 5016 attached thereto, the first conveyance control unit 143 conveys the syringe 501 to the needle removal unit 170. The needle removal control unit 154 removes the needle 5014 from the syringe 501. As a result, the needle 5014 is discarded into the first housing unit 70A. Further, the first conveyance control unit 143 conveys and discards the syringe 501 from which the needle 5014 has been removed, to the second housing unit 70B.

After power is restored, when the control unit 140 determines that the equipment being conveyed is a syringe 501 having the needle cap 5016 attached thereto, the control unit 140 performs control for prompting a user to collect the syringe 501. Specifically, the first conveyance control unit 143 directs the first claw part 111 or the second claw part 112 that holds the syringe 501, toward the central door 100 side, and then the control unit 140 releases the locked state of the central door 100. Accordingly, the user can open the central door 100 to take out the syringe 501. When the control unit 140 releases the locked state of the central door 100, the touch panel control unit 150 may display, on the touch panel 80, a notification image that prompts the user to take out the syringe 501 from the drug-mixing apparatus 1.

After power is restored, when the control unit 140 determines that the equipment being conveyed is a vial 502, the first conveyance control unit 143 conveys the vial 502 to the second housing unit 70B. The same holds true for the case in which the drug-mixing apparatus 1 does not include the needle removal unit 170.

### <Case of Including No Needle Removal Unit>

When the drug-mixing apparatus 1 does not include the needle removal unit 170, the control unit 140 may perform, for example, the following process.

After power is restored, when the control unit 140 determines that the equipment being conveyed is a syringe 501 having no needle cap 5016 attached thereto, the first conveyance control unit 143 conveys the syringe 501 to the cap removal unit 180. The control unit 140 controls the cap removal unit 180 to attach the needle cap 5016 to the needle 5014 attached to the syringe 501. After that, the first conveyance control unit 143 conveys and discards the syringe 501 having the needle cap 5016 attached thereto, to the waste box 70 having no first housing unit 70A attached thereto.

After power is restored, when the control unit 140 determines that the equipment being conveyed is a syringe 501 having the needle cap 5016 attached thereto, the control unit 140 performs control for prompting a user to collect the syringe 501. An example of the control has been described, and hence description thereof is omitted here.

### [Operation Example at Time of Removing Needle (Applying Vibration to Syringe)]

As described above, the drug-mixing apparatus 1 includes the needle removal unit 170 that removes the needle 5014 from the syringe 501. However, due to the presence of infusion between the needle 5014 and the barrel 5011, the needle 5014 is not completely removed from the barrel 5011 by the operation of the needle removal unit 170 described above, in some cases.

The drug-mixing apparatus 1 may include a vibration unit that applies vibration to the syringe 501 subjected to the operation of removing the needle 5014 by the needle removal unit 170. Accordingly, even when the infusion is present between the needle 5014 and the barrel 5011, the needle 5014 is more easily removed completely from the barrel 5011.

In the drug-mixing apparatus 1, the first conveyance unit 110 may function as a vibration unit. Specifically, the first conveyance unit 110 may apply vibration to the syringe 501 by lifting the syringe 501 subjected to the operation of removing the needle 5014 by the needle removal unit 170, from the needle removal unit 170, and then releasing the holding state of the syringe 501 to drop the syringe 501 onto the needle removal unit 170. Accordingly, vibration can be applied to the syringe 501 without adding a component for applying vibration to the syringe 501, and thus the needle 5014 can be more easily removed completely from the syringe 501. After applying vibration to the syringe 501, the first conveyance unit 110 holds the syringe 501 again to convey the syringe 501 to the waste box 70 (see FIG. 11). The first conveyance unit 110 may be an example of a holding unit that holds the syringe 501 subjected to the removal operation.

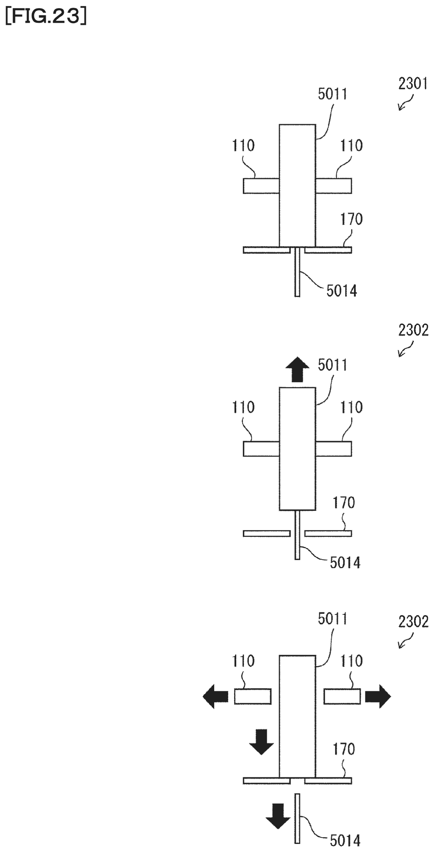

FIG. 23 is a schematic view for illustrating an outline of the operation of the first conveyance unit 110 as a vibration unit. Reference numeral 2301 in FIG. 23 indicates a state in which the needle 5014 remains on the syringe 501 after the operation of removing the needle 5014 by the needle removal unit 170. Even after causing the needle removal unit 170 to execute the operation of removing the needle 5014, when the second detection unit 178 (see FIG. 10) still detects the needle 5014 or the insertion hub 5019 (see FIG. 10), the needle removal control unit 154 determines that the needle 5014 has not been removed from the barrel 5011. When the needle removal control unit 154 determines that the needle 5014 has not been removed from the barrel 5011, the first conveyance unit 110 holds the syringe 501 as indicated by reference numeral 2301 in FIG. 23.

Then, the first conveyance unit 110 lifts the syringe 501 from the needle removal unit 170 as indicated by reference numeral 2302 in FIG. 23. After that, the first conveyance unit 110 releases the holding state of the syringe 501 to drop the syringe 501 onto the needle removal unit 170, as indicated by reference numeral 2303 in FIG. 23. As a result, the needle 5014 can be dropped from the syringe 501 due to the vibration generated in the syringe 501 when the syringe 501 collides with the needle removal unit 170.

When the syringe 501 is dropped, there is a possibility that the flange 5020 (see FIG. 10) of the insertion hub 5019 is caught on the edge of the insertion port 174A (see FIG. 10) of the needle removal unit 170, resulting in that the syringe 501 tilts. In this case, there is a possibility that the needle 5014 and the insertion hub 5019 are not inserted into the insertion port 174A. The first conveyance unit 110 may hold the syringe 501 again after dropping the syringe 501. Accordingly, even when the syringe 501 tilts, the tilt can be corrected. Thus, even when the needle 5014 and the insertion hub 5019 are caught on the edge of the insertion port 174A, the state of being caught can be released, and hence the needle 5014 and the insertion hub 5019 can be inserted into the insertion port 174A.

The first conveyance unit 110 may apply vibration to all syringes 501 after the removal operation by the needle removal unit 170. Alternatively, as described above, the first conveyance unit 110 may apply vibration only to the syringe 501 from which the needle 5014 has not been removed, after the removal operation by the needle removal unit 170. For example, when the second detection unit 178 still detects the needle 5014 after the needle removal operation by the needle removal unit 170, the first conveyance unit 110 may apply vibration to the syringe 501, assuming that the needle 5014 has not been removed from the syringe 501.

Further, the drug-mixing apparatus 1 may include a vibration unit different from the first conveyance unit 110, which applies vibration to the syringe 501. For example, the drug-mixing apparatus 1 may include a vibration unit that applies vibration to the syringe 501 by applying an impact to the side surface of the barrel 5011.

### [Operation Example at Time of Discarding Syringe]

As described above, the first conveyance unit 110 conveys the syringe 501 from which the needle 5014 has been removed by the needle removal unit 170, to the waste box 70. At this time, the first conveyance unit 110 may hold the syringe 501 under a state of being in abutment against the flange 5013 of the syringe 501, and may discard the syringe 501 into the waste box 70 by releasing the holding state. The first conveyance unit 110 may be an example of a holding unit that holds the syringe 501 used in the drug-mixing operation.

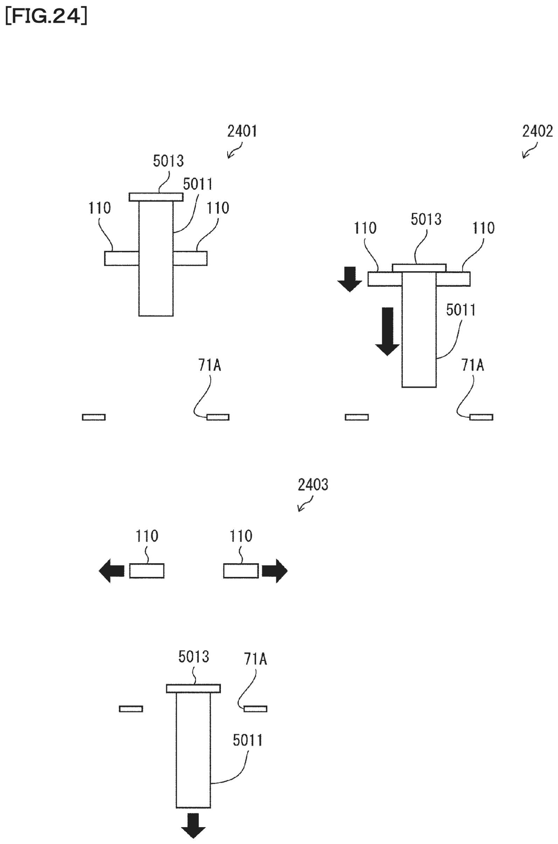

FIG. 24 is a schematic view for illustrating an outline of an operation in which the first conveyance unit 110 discards the syringe 501 into the waste box 70. As indicated by reference numeral 2401 in FIG. 24, the first conveyance unit 110 conveys the syringe 501 from which the needle 5014 has been removed by the needle removal unit 170, to a position directly above an opening portion 71A of the waste box 70. At this point in time, the first conveyance unit 110 may hold the syringe 501 under a state of being in abutment against the vicinity of the center of the barrel 5011.

The waste box 70 is an example of a housing unit in which the syringe 501 used in the drug-mixing operation is allowed to be discarded. The opening portion 71A may be an opening portion of the second housing unit 70B, and the syringe 501 used in the drug-mixing operation may be discarded into the second housing unit 70B.

As indicated by reference numeral 2402 in FIG. 24, the first conveyance unit 110 descends toward the opening portion 71A. At this time, the first conveyance unit 110 temporarily releases the holding state of the syringe 501, to thereby drop the syringe 501 relative to the first conveyance unit 110. At this time, the syringe 501 moves downward farther than the first conveyance unit 110. Further, with this movement, the first claw part 111 or the second claw part 112 (see FIG. 14) of the first conveyance unit 110 that holds the syringe 501 abuts against the flange 5013. The first conveyance unit 110 re-holds the syringe 501 under a state in which the flange 5013 is in abutment against the first conveyance unit 110. Accordingly, the first conveyance unit 110 holds the syringe 501 under a state of being in abutment against the flange 5013. After that, as indicated by reference numeral 2403 in FIG. 24, the first conveyance unit 110 releases the holding state of the syringe 501 again, to thereby discard the syringe 501 into the waste box 70.

When the first conveyance unit 110 releases the holding of the syringe 501 while holding the barrel 5011 at a position away from the flange 5013 (for example, in the vicinity of the center of the barrel 5011), there is a possibility that the syringe 501 may drop under a tilted state. Assuming this possibility, the size of the opening portion 71A is secured to an extent that allows the syringe 501 to pass through the opening portion 71A even under a state in which the syringe 501 is tilted. As described above, the first conveyance unit 110 releases the holding state of the syringe 501 under a state of being in abutment against the flange 5013, to discard the syringe 501 into the waste box 70, and thus the tilt of the syringe 501 is likely to be smaller at the time of discarding the syringe 501 into the waste box 70. Accordingly, the size of the opening portion 71A of the waste box 70 that receives the discarded syringe 501 can be reduced.

However, the first conveyance unit 110 may hold the syringe 501 under a state of being in abutment against the flange 5013, at the time of conveying the syringe 501 from which the needle 5014 has been removed, to a position directly above the opening portion 71A of the waste box 70. In this case, after conveying the syringe 501 to the position directly above the opening portion 71A of the waste box 70, the first conveyance unit 110 may immediately release the holding state of the syringe 501 to discard the syringe 501 into the waste box 70.

Further, the first conveyance unit 110 may descend to a position at which a distal end of the held syringe 501 is lower than the opening portion 71A, and then may release the holding state of the syringe 501. Accordingly, in the case in which the size of the opening portion 71A is set to be relatively small, even when the syringe 501 tilts after the release of the holding state of the syringe 501 by the first conveyance unit 110, the syringe 501 can be discarded into the waste box 70.

### [Example of Manually Removing Needle]

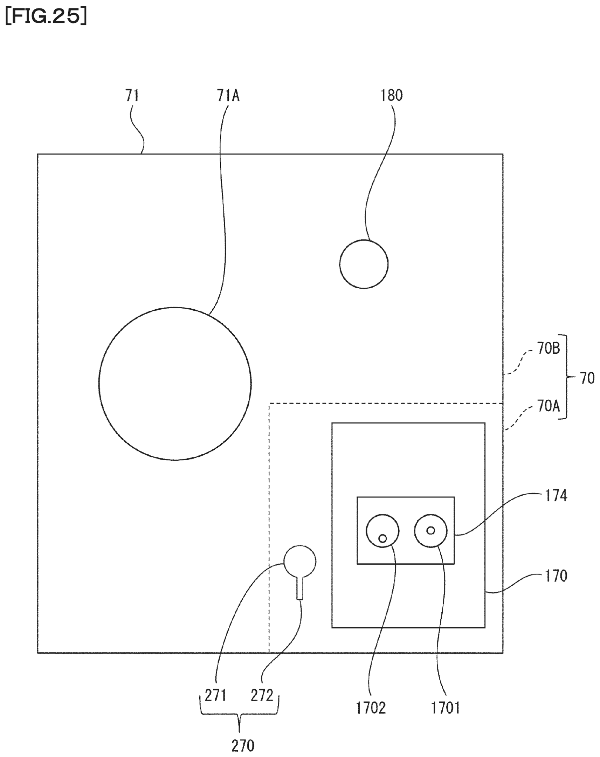

FIG. 25 is a plan view for illustrating an example of a partition member 71 provided on the upper side of the waste box 70. The partition member 71 is a member having a substantially flat plate shape, which covers the entire upper side of the waste box 70. The partition member 71 partitions a drug-mixing space in which conveyance of equipment such as the syringe 501 and a drug-mixing operation are performed, and a space in which the waste box 70 is placed.

As illustrated in FIG. 25, the needle removal unit 170 and the cap removal unit 180 are arranged on the partition member 71. Further, the partition member 71 has the opening portion 71A through which the syringe 501 and the vial 502 used in the drug-mixing operation are allowed to be discarded into the waste box 70. The opening portion 71A may be provided with a shutter that is normally in a closed state and opens only at the time of discarding the syringe 501 and the vial 502 into the waste box 70.

The cap removal unit 180 may be located above the second housing unit 70B. Further, the needle cap 5016 (see FIG. 6) removed by the cap removal unit 180 may be dropped from the cap removal unit 180 into the second housing unit 70B as described later, instead of being conveyed to the opening portion 71A by the first conveyance unit 110 as described later.

The partition member 71 may be further provided with a needle removal unit 270. The needle removal unit 270 is an opening portion through which a user is allowed to manually remove the needle 5014 from the syringe 501 to discard the needle 5014 into the waste box 70. As illustrated in FIG. 25, the needle removal unit 270 includes an introduction part 271 and a needle removal part 272. The needle removal unit 270 may be located above the first housing unit 70A, similarly to the needle removal unit 170.

The introduction part 271 is an opening through which the needle 5014 of the syringe 501 is allowed to be introduced into the waste box 70. The introduction part 271 has a size that allows the flange 5020 (see FIG. 10) of the insertion hub 5019 to pass through the introduction part 271. The needle removal part 272 is a portion with which the needle 5014 is allowed to be removed from the syringe 501. The needle removal part 272 has a size that allows the barrel tip 5017 (see FIG. 7) of the syringe 501 to pass through the needle removal part 272, but does not allow the flange 5020 to pass through the needle removal part 272.

At the time of removing the needle 5014 from the syringe 501 by the needle removal unit 270, a user introduces the needle 5014 from the introduction part 271 to the waste box 70 side so that the height of the barrel tip 5017 becomes equal to the height of the partition member 71. After that, the user moves the syringe 501 so that the barrel tip 5017 moves from the introduction part 271 to the needle removal part 272, and further pulls the syringe 501 upward. Accordingly, the flange 5020 is caught on the needle removal part 272 so that the needle 5014 is pulled out from the barrel tip 5017 to be discarded into the waste box 70.

### [Example of Operation of Pressing Needle Cap against Syringe]

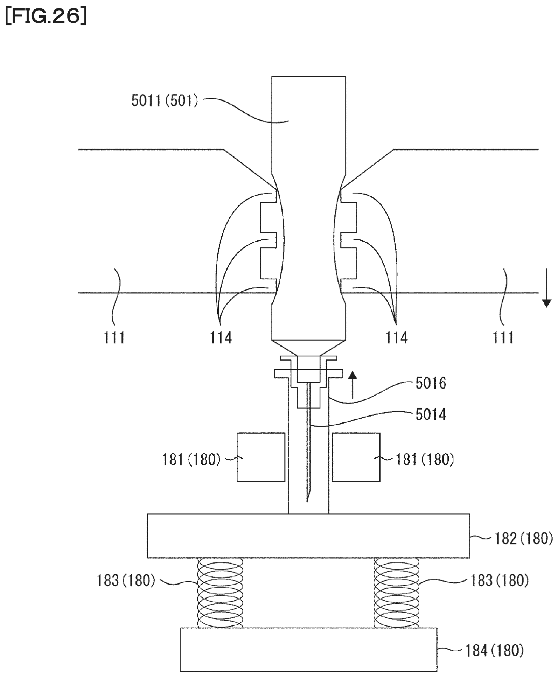

FIG. 26 is a schematic view for illustrating an outline of an operation of pressing the needle cap 5016 of the syringe 501 by the first conveyance unit 110. As illustrated in FIG. 26, the cap removal unit 180 includes a cap holding unit 181, a first pedestal 182, a spring 183 that is an example of an elastic member, and a second pedestal 184.

The cap holding unit 181 holds the needle cap 5016 attached to the needle 5014 of the syringe 501, or the needle cap 5016 removed from the syringe 501. The first pedestal 182 is a pedestal against which the needle cap 5016 is to be pressed. The second pedestal 184 is a pedestal that supports the first pedestal 182. The spring 183 is arranged between the first pedestal 182 and the second pedestal 184. The first pedestal 182 is movable in the up-down direction by the spring 183.

Further, the first pedestal 182, the spring 183, and the second pedestal 184 may be provided so as to be movable in the horizontal direction. The first pedestal 182, the spring 183, and the second pedestal 184 may be movable between a first position directly below the cap holding unit 181 and a second position other than the position directly below the cap holding unit 181.

The first conveyance control unit 143 inserts the first conveyance unit 110 into the cap removal unit 180 under a state in which the first claw part 111 holds the barrel 5011. At this time, the first conveyance control unit 143 moves the first conveyance unit 110 toward the first pedestal 182. The first pedestal 182 is supported by the second pedestal 184 via the spring 183, and hence the needle cap 5016 is pressed against the syringe 501 side (barrel 5011 side) with an appropriate strength regardless of the length of the needle 5014 and the needle cap 5016. The needle cap 5016 is pressed against the barrel 5011, and thus the needle 5014 inside the needle cap 5016 is pressed against the barrel 5011 to be firmly held by the barrel 5011.

After that, the first conveyance control unit 143 moves the first conveyance unit 110 upward to separate the needle cap 5016 from the first pedestal 182. Under this state, the control unit 140 causes the cap holding unit 181 to hold the needle cap 5016. Then, the first conveyance control unit 143 moves the first conveyance unit 110 away from the cap holding unit 181 under a state in which the first conveyance unit 110 holds the barrel 5011 and the cap holding unit 181 holds the needle cap 5016. With this movement, the needle cap 5016 is removed from the needle 5014 of the syringe 501.

The control unit 140 maintains a state in which the needle cap 5016 removed from the needle 5014 is held by the cap holding unit 181, until the drug-mixing-unit control unit 144 starts an operation of puncturing the stopper 5021 (see FIG. 18) of the vial 502 with the needle 5014 of the syringe 501. Accordingly, when the puncture of the stopper 5021 with the needle 5014 is canceled after the needle cap 5016 is removed from the needle 5014 of the syringe 501, the syringe 501 can be reused by reattaching the needle cap 5016 held by the cap holding unit 181, to the needle 5014.

When the first pedestal 182, the spring 183, and the second pedestal 184 are movable, after the needle cap 5016 is removed by the movement of the first conveyance unit 110, the control unit 140 moves the first pedestal 182, the spring 183, and the second pedestal 184 from the above-mentioned first position to the above-mentioned second position. After that, the control unit 140 releases the holding of the needle cap 5016 by the cap holding unit 181. Accordingly, the needle cap 5016 is discarded into the waste box 70 located below the cap removal unit 180.

Meanwhile, when the first pedestal 182, the spring 183, and the second pedestal 184 are not movable, as described above, the first conveyance unit 110 holds the needle cap 5016 held by the cap holding unit 181, and then, the cap holding unit 181 releases its holding. Then, the first conveyance unit 110 conveys and discards the needle cap 5016 to the waste box 70.

Further, the cap removal unit 180 is not always required to include the first pedestal 182, the spring 183, and the second pedestal 184. In this case, the first conveyance control unit 143 moves the first conveyance unit 110 toward the cap removal unit 180 side (insertion direction of the needle 5014) under a state in which the first claw part 111 holds the barrel 5011 and the cap holding unit 181 holds the needle cap 5016. The position of the cap holding unit 181 is fixed, and hence the needle cap 5016 does not move by this movement. Accordingly, with this movement, the needle cap 5016 is pressed against the syringe 501 side (barrel 5011 side). Also in this case, the needle cap 5016 is pressed against the barrel 5011, and thus the needle 5014 inside the needle cap 5016 is pressed against the barrel 5011 to be firmly held by the barrel 5011.

### [Supplementary Notes]

The present invention is not limited to the embodiments described above, and various modifications are possible within the scope of claims. Embodiments obtained by appropriately combining technical means disclosed in different embodiments are also included in the technical scope of the present invention.

### List of numeral references

FIG. 4
   - 10: SYRINGE SHELF
   - 11: EQUIPMENT HOLDING UNIT
   - 12: EQUIPMENT CONVEYANCE UNIT
   - 13: OBJECT DETECTION UNIT
   - 14: SYRINGE DETECTION UNIT
   - 15: NEEDLE DETECTION UNIT
   - 16: SYRINGE SIDE DOOR
   - 17: BARREL DETECTION UNIT
   - 18: BARREL TIP DETECTION UNIT
   - 20: VIAL SHELF
   - 21: EQUIPMENT HOLDING UNIT
   - 22: EQUIPMENT CONVEYANCE UNIT
   - 23: OBJECT DETECTION UNIT
   - 24: VIAL DETECTION UNIT
   - 25: FIRST READING UNIT
   - 26: ROLLER DRIVING UNIT
   - 27: VIAL SIDE DOOR
   - 30: INFUSION SHELF
   - 31: PUSHING-IN UNIT
   - 34: INFUSION SIDE DOOR
   - 37: SHUTTER
   - 40: DRUG-MIXING UNIT
   - 41: SYRINGE HOLDING UNIT
   - 42: VIAL HOLDING UNIT
   - 43: INFUSION CONTAINER HOLDING UNIT
   - 44: BASE
   - 50: PRINTING/INSPECTION UNIT
   - 80: TOUCH PANEL
   - 110: FIRST CONVEYANCE UNIT
   - 120: SECOND CONVEYANCE UNIT
   - 121: SUCTION UNIT
   - 122: THIRD READING UNIT
   - 140: CONTROL UNIT
   - 141: SYRINGE CONVEYANCE CONTROL UNIT
   - 142: VIAL CONVEYANCE CONTROL UNIT
   - 143: FIRST CONVEYANCE CONTROL UNIT
   - 144: DRUG-MIXING-UNIT CONTROL UNIT
   - 145: SECOND CONVEYANCE CONTROL UNIT
   - 147: PUSHING-IN CONTROL UNIT
   - 148: SHUTTER CONTROL UNIT
   - 149: PRINTING/INSPECTION UNIT CONTROL UNIT
   - 150: TOUCH PANEL CONTROL UNIT
   - 151: DETERMINATION UNIT
   - 154: NEEDLE REMOVAL CONTROL UNIT
   - 170: NEEDLE REMOVAL UNIT
   - 180: CAP REMOVAL UNIT
   - 200: STORAGE UNIT
FIG. 13
   - 18a: FIRST BARREL TIP DETECTION UNIT
   - 18b: SECOND BARREL TIP DETECTION UNIT
   - 18c: THIRD BARREL TIP DETECTION UNIT
FIG. 20
   - S1: WITHDRAW INFUSION FROM INFUSION CONTAINER
   - S2: INJECT PART OF WITHDRAWN INFUSION INTO VIAL
   - S3: WITHDRAW INFUSION HAVING POWDERED DRUG DISSOLVED THEREIN, FROM VIAL
   - S4: MIX INFUSION HAVING POWDERED DRUG DISSOLVED THEREIN, WITH REMAINING INFUSION IN SYRINGE
   - S5: PROCESS EXECUTED PREDETERMINED NUMBER OF TIMES?
   - S6: INJECT PART OF WITHDRAWN INFUSION INTO VIAL
   - S7: TILT VIAL
   - S8: WITHDRAW PREDETERMINED AMOUNT OF INFUSION HAVING POWDERED DRUG DISSOLVED THEREIN, FROM VIAL
   - S9: MIX INFUSION HAVING POWDERED DRUG DISSOLVED THEREIN, WITH REMAINING INFUSION IN SYRINGE
   - S10: PREDETERMINED TIME ELAPSED?
   - S11: RETURN VIAL TO ORIGINAL POSITION
   - S12: WITHDRAW INFUSION HAVING POWDERED DRUG DISSOLVED THEREIN, FROM VIAL
FIG. 21
   - (1): INFUSION
   - (2): POWDERED DRUG
FIG. 22
   - (1): BUBBLES
   - (2): INFUSION

## Claims

1. A drug-mixing apparatus, comprising:
a drug-mixing unit configured to execute a drug-mixing operation of mixing a drug housed in a drug container into an infusion housed in an infusion container, using a syringe having a needle attached thereto; and
a needle removal part configured to remove the needle from the syringe used in the drug-mixing operation, after the drug-mixing operation has ended.

2. The drug-mixing apparatus according to claim 1, further comprising:
a first housing unit configured to house a needle; and
a second housing unit configured to house a syringe having no needle attached thereto,
wherein the needle removed from the syringe by the needle removal part is discarded into the first housing unit, and
wherein the syringe from which the needle has been removed by the needle removal part is discarded into the second housing unit.

3. The drug-mixing apparatus according to claim 1 or 2, wherein the needle removal part includes:
a first needle removal part configured to remove a needle attached to a syringe of a centric tip type as the syringe, from the syringe; and
a second needle removal part configured to remove a needle attached to a syringe of an eccentric tip type as the syringe, from the syringe.

4. The drug-mixing apparatus according to claim 3, further comprising:
a determination unit configured to determine whether the syringe is a syringe of a centric tip type or a syringe of an eccentric tip type; and
a conveyance unit configured to convey the syringe to the first needle removal part or the second needle removal part based on a determination result obtained from the determination unit.

5. The drug-mixing apparatus according to any one of claims 1 to 4,
wherein the needle is attached to an insertion hub having a flange, and is attached to the syringe via the insertion hub,
wherein the needle removal part includes:
a member having an insertion port into which the insertion hub and the needle that have been attached to the syringe are allowed to be inserted;
a claw part, which rotates about a rotation axis extending in a direction perpendicular to an insertion direction of the needle, and is allowed to abut against the flange of the insertion hub inserted from the insertion port; and
a detection unit configured to detect the needle or the insertion hub that has been inserted from the insertion port, and
wherein, when the detection unit detects the needle or the insertion hub, the claw part is brought into abutment against the flange, and the insertion hub and the needle are removed from the syringe by applying a force to the flange in the insertion direction of the needle.

6. A drug-mixing apparatus, comprising:
a drug-mixing unit configured to dissolve, based on a prescription data group including a plurality of pieces of prescription data, a powdered drug housed in a drug container, with an infusion, and execute a drug-mixing operation of mixing the infusion having the powdered drug dissolved therein, into an infusion housed in an infusion container, using a syringe; and
an equipment removal unit configured to remove the syringe, the drug container, and the infusion container that have been held by the drug-mixing unit, from the drug-mixing unit,
wherein, in the drug-mixing operation, the drug-mixing unit waits for a predetermined time after injecting the infusion into the drug container, and then withdraws the infusion from the drug container, and
wherein, when a second drug-mixing operation for second prescription data is to be executed after a first drug-mixing operation for first prescription data, after the equipment removal unit removes the syringe, the drug container, and the infusion container being used in the first drug-mixing operation, from the drug-mixing unit during the waiting for the predetermined time in the first drug-mixing operation, the drug-mixing unit starts the second drug-mixing operation.

7. The drug-mixing apparatus according to claim 6,
wherein the equipment removal unit includes a plurality of equipment holding units, and
wherein part of the plurality of equipment holding units is configured to hold at least one of the syringe, the drug container, or the infusion container being used in the first drug-mixing operation, until the drug-mixing unit resumes the first drug-mixing operation.

8. The drug-mixing apparatus according to claim 7,
wherein the equipment removal unit includes:
a first equipment removal unit configured to remove the syringe and the drug container from the drug-mixing unit; and
a second equipment removal unit configured to remove the infusion container from the drug-mixing unit,
wherein part of the plurality of equipment holding units included in the first equipment removal unit is configured to hold at least one of the syringe or the drug container being used in the first drug-mixing operation, and
wherein part of the plurality of equipment holding units included in the second equipment removal unit is configured to hold the infusion container being used in the first drug-mixing operation.

9. The drug-mixing apparatus according to any one of claims 6 to 8,
wherein, even when the predetermined time has elapsed during the execution of the second drug-mixing operation, the drug-mixing unit continues the second drug-mixing operation,
wherein, after the drug-mixing unit has completed the second drug-mixing operation, the equipment removal unit delivers the syringe, the drug container, and the infusion container, which have been removed from the drug-mixing unit, to the drug-mixing unit, and
wherein, after the equipment removal unit has delivered the syringe, the drug container, and the infusion container to the drug-mixing unit, the drug-mixing unit resumes the first drug-mixing operation.

10. The drug-mixing apparatus according to any one of claims 6 to 9,
wherein, in the prescription data group, the plurality of pieces of prescription data are grouped, and
wherein, when the first prescription data and the second prescription data belong to different groups, the equipment removal unit removes the syringe, the drug container, and the infusion container, which have been used in the first drug-mixing operation, from the drug-mixing unit after the first drug-mixing operation has ended.

11. A drug-mixing apparatus, which is configured to execute a drug-mixing operation of dissolving a powdered drug housed in a drug container, with an infusion, and mixing the infusion having the powdered drug dissolved therein, into an infusion housed in an infusion container,
wherein, when part of the infusion having the powdered drug dissolved therein is to be withdrawn from the drug container, after executing a dissolving operation of dissolving the powdered drug in the infusion, the drug-mixing apparatus withdraws the infusion having the powdered drug dissolved therein, from the drug container after a first predetermined time has elapsed, and
wherein, when all of the infusion having the powdered drug dissolved therein is to be withdrawn from the drug container, after executing the dissolving operation, the drug-mixing apparatus withdraws the infusion having the powdered drug dissolved therein, from the drug container after a second predetermined time shorter than the first predetermined time has elapsed.

12. The drug-mixing apparatus according to claim 11, wherein an amount of the powdered drug contained in part or all of the infusion having the powdered drug dissolved therein, which is to be withdrawn from the drug container, is a prescribed amount.

13. The drug-mixing apparatus according to claim 11 or 12, wherein the first predetermined time and the second predetermined time are set for each of a plurality of types of powdered drugs that are allowed to be handled in the drug-mixing apparatus.

14. A drug-mixing apparatus, comprising:
a housing unit into which a syringe used in a drug-mixing operation of mixing a drug housed in a drug container into an infusion housed in an infusion container is allowed to be discarded; and
a holding unit, which is configured to hold the syringe under a state of being in abutment against a flange of the syringe, and to discard the syringe into the housing unit by releasing a state of the holding.
